(19) Europäisches Patentamt
European Patent Office
Office européen des brevets

(11) Veröffentlichungsnummer : **0 431 390 B1**

(12) # EUROPÄISCHE PATENTSCHRIFT

(45) Veröffentlichungstag der Patentschrift : **12.01.94 Patentblatt 94/02**

(51) Int. Cl.$^5$ : **C07D 249/14,** C07D 413/04, A01N 43/653, A01N 43/90

(21) Anmeldenummer : **90122139.0**

(22) Anmeldetag : **20.11.90**

(54) **Substituierte Triazolinone.**

(30) Priorität : **02.12.89 DE 3939952**

(43) Veröffentlichungstag der Anmeldung : **12.06.91 Patentblatt 91/24**

(45) Bekanntmachung des Hinweises auf die Patenterteilung : **12.01.94 Patentblatt 94/02**

(84) Benannte Vertragsstaaten : **BE CH DE FR GB IT LI NL**

(56) Entgegenhaltungen :
**EP-A- 0 070 089**
**DE-A- 2 537 973**
**DE-A- 3 722 821**
**DE-A- 3 729 070**
**US-A- 4 846 875**

(73) Patentinhaber : **BAYER AG**
**D-51368 Leverkusen (DE)**

(72) Erfinder : **Findeisen, Kurt, Dr.**
**Dünfelder Strasse 28**
**W-5090 Leverkusen 1 (DE)**
Erfinder : **Santel, Hans-Joachim, Dr.**
**Grünstrasse 9a**
**W-5090 Leverkusen 1 (DE)**
Erfinder : **Lürssen, Klaus, Dr.**
**August-Kierspel-Strasse 145**
**W-5060 Bergisch-Gladbach 2 (DE)**
Erfinder : **Schmidt, Robert R., Dr.**
**Im Waldwinkel 110**
**W-5060 Bergisch Gladbach 2 (DE)**

**EP 0 431 390 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue substituierte Triazolinone, mehrere Verfahren zu ihrer Herstellung und ihre Verwendung als Herbizide.

Es wurden neue substituierte Triazolinone der allgemeinen Formel (I)

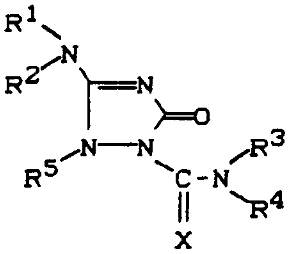

(I)

in welcher

$R^1$ und $R^2$ unabhängig voneinander jeweils für Alkyl, Alkenyl, Alkinyl, Halogenalkyl, Halogenalkenyl, Halogenalkinyl, Cyanalkyl, Alkoxyalkyl, Alkoxy, Cycloalkyl, Cycloalkylalkyl, für jeweils gegebenenfalls substituiertes Aryl, Aralkyl oder Heteroaryl stehen oder gemeinsam mit dem Stickstoffatom, an welches sie gebunden sind, für einen gegebenenfalls substituierten Heterocyclus stehen,

$R^3$ und $R^4$ unabhängig voneinander jeweils für Wasserstoff, Alkyl, Alkenyl, Alkinyl, Halogenalkyl, Halogenalkenyl, Halogenalkinyl, Cyanalkyl, Hydroxyalkyl, Alkoxyalkyl, Alkylaminoalkyl, Dialkylaminoalkyl, Alkoxycarbonylalkyl, Alkoxycarbonylalkenyl, für jeweils gegebenenfalls substituiertes Cycloalkyl, Cycloalkylalkyl, Cycloalkenyl oder Cycloalkenylalkyl, für gegebenenfalls substituiertes Heterocyclylalkyl, für jeweils gegebenenfalls substituiertes Aralkyl, Aroyl oder Aryl, für Alkoxy, Alkenyloxy, Alkinyloxy, Aralkyloxy oder Aryloxy stehen oder gemeinsam mit dem Stickstoffatom, an welches sie gebunden sind, für einen gegebenenfalls substituierten Heterocyclus stehen,

$R^5$ für Alkyl oder Cycloalkyl steht und

X für Sauerstoff oder Schwefel steht,

gefunden.

Weiter wurde gefunden, daß man die neuen substituierten Triazolinone der allgemeinen Formel (I) erhält, wenn man

a) Triazolinone der allgemeinen Formel (II)

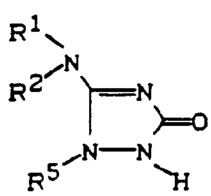

(II)

in welcher

$R^1$, $R^2$ und $R^5$ die oben angegebene Bedeutung haben,

und/oder Tautomere dieser Verbindungen, d.h. die entsprechenden Hydroxytriazole,

mit Iso(thio)cyanaten der allgemeinen Formel (III)

$$R^4\text{-N=C=X} \qquad (III)$$

in welcher

$R^4$ und X die oben angegebene Bedeutung haben,

gegebenenfalls in Gegenwart eines Reaktionshilfsmittels und gegebenenfalls in Gegenwart eines Verdünnungsmittels umsetzt, oder wenn man

b) Chlorcarbonyl-triazolinone der allgemeinen Formel (IV)

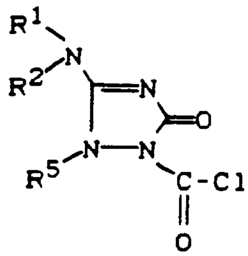

(IV)

in welcher
$R^1$, $R^2$ und $R^5$ die oben angegebene Bedeutung haben,
mit Aminen der allgemeinen Formel (V)

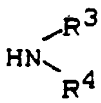

(V)

in welcher
$R^3$ und $R^4$ die oben angegebene Bedeutung haben,
gegebenenfalls in Gegenwart eines Säurebindemittels und gegebenenfalls in Gegenwart eines Verdünnungsmittels umsetzt.

Schließlich wurde gefunden, daß die neuen substituierten Triazolinone der allgemeinen Formel (I) interessante herbizide Eigenschaften aufweisen.

In den allgemeinen Formeln bedeutet Halogen Fluor, Chlor, Brom und Iod, vorzugsweise Fluor, Chlor oder Brom, insbesondere Fluor oder Chlor.

Gegebenenfalls substituierte Aryl-Gruppen wie beispielsweise Aryl, Aralkyl, Aryloxy können einen oder mehrere, vorzugsweise 1 bis 5, insbesondere 1 bis 3 und besonders bevorzugt 1 oder 2 gleiche oder verschiedene Substituenten tragen.

In arylhaltigen Resten steht Aryl vorzugsweise für Phenyl oder Naphthyl.

Die Reste $R^1$ und $R^2$ bzw. $R^3$ und $R^4$ stehen für den Fall, daß sie gemeinsam mit dem Stickstoffatom, an welches sie gebunden sind, einen Heterocyclus bilden, vorzugsweise für einen gegebenenfalls einfach, zweifach oder dreifach, gleich oder verschieden substituierten Heterocyclus der Formel

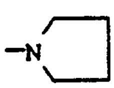 ; 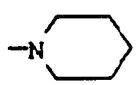 ; 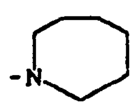 ; 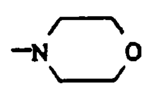 ;

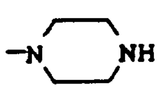 ; 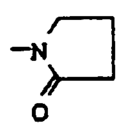 ; 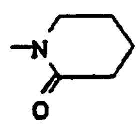 ; 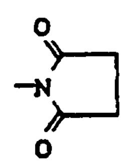 ;

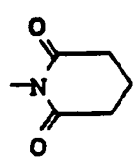 ; 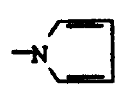 oder 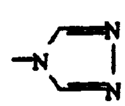 ,

wobei als Substituenten ausgewählt sind:

Halogen sowie jeweils geradkettiges oder verzweigtes Alkyl oder Halogenalkyl mit jeweils 1 bis 4 Kohlenstoffatomen und gegebenenfalls 1 bis 9, vorzugsweise 1 bis 5, insbesondere 1 bis 3 gleichen oder verschiedenen Halogenatomen. Besonders bevorzugt sind als Substituenten Methyl, Ethyl, n- oder i-Propyl, Chlor und Trifluormethyl ausgewählt.

Der Rest Heterocyclus in der Bedeutung von Heterocyclylalkyl für $R^3$ und $R^4$ steht vorzugsweise für die folgenden Heterocyclen:

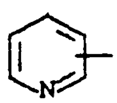 ; 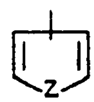 ; 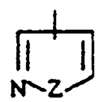 ; 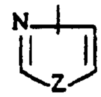 ;

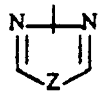 ; 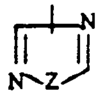 ; 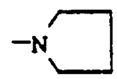 ; 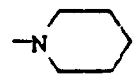 ;

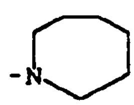 ; 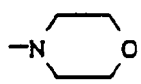 oder 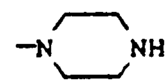

wobei Z jeweils für Sauerstoff oder Schwefel steht.

Alkyl in der Bedeutung von Heterocyclylalkyl steht beispielsweise für Methyl, Ethyl, n- oder iso-Propyl, n-, iso-, sec. oder tert.-Butyl.

Der Heterocyclus in der Bedeutung von Heterocyclylalkyl kann einfach oder mehrfach, vorzugsweise einfach bis dreifach, insbesondere einfach oder zweifach gleich oder verschieden durch jeweils geradkettiges oder verzweigtes Alkyl oder Halogenalkyl mit 1 bis 4, vorzugsweise 1 oder 2 Kohlenstoffatomen und 1 bis 9, vorzugsweise 1 bis 5, insbesondere 1 bis 3 gleichen oder verschiedenen Halogenatomen oder jeweils zweifach verknüpftes Alkandiyl oder Alkendiyl mit bis zu 4 Kohlenstoffatomen substituiert sein. Insbesondere sind als Substituenten Methyl und Ethyl ausgewählt.

Bevorzugt sind Verbindungen der Formel (I), bei welchen

$R^1$ und $R^2$ unabhängig voneinander für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl, Alkoxyalkyl oder Alkoxy mit jeweils 1 bis 6 Kohlenstoffatomen in den einzelnen Alkylteilen, für Cycloalkyl mit 3 bis 7 Kohlenstoffatomen, für Cycloalkylalkyl mit 3 bis 7 Kohlenstoffatomen im Cycloalkylteil und 1 bis 6 Kohlenstoffatomen im Alkylteil oder für jeweils gegebenenfalls einfach oder mehrfach, gleich oder verschieden substituiertes Aralkyl mit 6 bis 10 Kohlenstoffatomen im Arylteil und 1 bis 6 Kohlenstoffatomen im Alkylteil, Aryl mit 6 bis 10 Kohlenstoffatomen oder Heteroaryl mit 2 bis 9 Kohlenstoffatomen und 1 bis 3 Heteroatomen, insbesondere Stickstoff, Sauerstoff und/oder Schwefel stehen, wobei als Substituenten jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen oder

$R^1$ und $R^2$ gemeinsam mit dem Stickstoffatom, an welches sie gebunden sind, für einen gegebenenfalls einfach oder mehrfach gleich oder verschieden substituierten fünf- bis zehngliedrigen Heterocyclus stehen, der gegebenenfalls 1 bis 2 weitere Heteroatome, insbesondere Stickstoff, Sauerstoff und/oder Schwefel enthalten kann, wobei als Substituenten infrage kommen: Halogen sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen sowie 1 bis 2 Oxo- oder Thionogruppen,

$R^3$ und $R^4$ unabhängig voneinander für Wasserstoff, für jeweils geradkettiges oder verzweigtes Alkyl mit

1 bis 18 Kohlenstoffatomen, Alkenyl mit 2 bis 8 Kohlenstofftomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, Alkoxyalkyl, Alkoxycarbonylalkyl oder Alkoxycarbonylalkenyl mit jeweils bis zu 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen oder für jeweils gegebenenfalls einfach oder mehrfach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im Alkylteil stehen, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenene Halogenatomen oder jeweils zweifach verknüpftes Alkandiyl bzw. Alkendiyl mit jeweils bis zu 4 Kohlenstoffatomen; außerdem $R^3$ und $R^4$ unabhängig voneinander für im Heterocyclylteil gegebenenfalls einfach oder mehrfach, gleich oder verschieden substituiertes Heterocyclylalkyl mit 1 bis 6 Kohlenstoffatomen im geradkettigen oder verzweigten Alkylteil und 1 bis 9 Kohlenstoffatomen sowie 1 bis 3 Heteroatomen -insbesondere Stickstoff, Sauerstoff und/oder Schwefel - im Heterocyclylteil stehen, wobei als Substituenten infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen; außerdem $R^3$ und $R^4$ unabhängig voneinander für jeweils geradkettiges oder verzweigtes Alkoxy mit 1 bis 8 Kohlenstoffatomen, Alkenyloxy mit 2 bis 8 Kohlenstoffatomen oder Alkinyloxy mit 2 bis 8 Kohlenstoffatomen oder für jeweils gegebenenfalls einfach oder mehrfach, gleich oder verschieden substituiertes Aralkyl, Aralkyloxy, Aryloxy, Aroyl oder Aryl mit jeweils 6 bis 10 Kohlenstoffatomen im Arylteil und gegebenenfalls 1 bis 6 Kohlenstoffatomen im Alkylteil stehen, wobei als Arylsubstituenten jeweils infrage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen und gegebenenfalls 1 bis 9 gleichen oder verschiedenen Halogenatomen, Cycloalkyl mit 3 bis 6 Kohlenstoffatomen oder Phenoxy und wobei als Alkylsubstituenten gegebenenfalls infrage kommen: Halogen oder Cyano, oder

$R^3$ und $R^4$ gemeinsam mit dem Stickstoffatom, an welches sie gebunden sind, für einen gegebenenfalls einfach oder mehrfach, gleich oder verschieden substituierten fünf- bis zehngliedrigen Heterocyclus stehen, der gegebenenfalls 1 bis 2 weitere Heteroatome, insbesondere Stickstoff, Sauerstoff und/oder Schwefel, enthalten kann, wobei als Substituenten infrage kommen: Halogen sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen sowie 1 bis 2 Oxo- oder Thionogruppen,

$R^5$ für geradkettiges oder verzweigtes Alkyl mit 1 bis 8 Kohlenstoffatomen oder Cycloalkyl mit 3 bis 8 Kohlenstoffatomen steht und

X für Sauerstoff oder Schwefel steht.

Besonders bevorzugt sind die Verbindungen der Formel (I), bei welchen

$R^1$ und $R^2$ unabhängig voneinander jeweils für Methyl, Ethyl, n- oder i-Propyl, n-, i-, s- oder t-Butyl, n- oder i-Pentyl, Allyl, Propargyl, für jeweils geradkettiges oder verzweigtes Halogenalkyl mit 1 bis 4 Kohlenstoffatomen, Halogenalkenyl mit 3 bis 6 Kohlenstoffatomen oder Halogenalkinyl mit 3 bis 6 Kohlenstoffatomen und jeweils 1 bis 9 gleichen oder verschiedenen Halogenatomen, für Cyanethyl, Methoxymethyl, Methoxyethyl, Dimethoxyethyl, Methoxy, Ethoxy, für Cyclopropyl, Cyclopropylmethyl, Cyclopentyl, Cyclohexyl, Cyclohexylmethyl, Cyclohexylethyl, Cyclopentylmethyl oder für jeweils gegebenenfalls ein- bis dreifach, gleich oder verschieden substituiertes Benzyl, Phenylethyl oder Phenyl stehen, wobei als Substituenten infrage kommen: Fluor, Chlor, Brom, Cyano, Nitro, Methyl, Ethyl, n- oder i-Propyl, n-, i-, s-oder t-Butyl, Methoxy, Ethoxy, Methylthio, Trifluormethyl, Trifluormethoxy oder Trifluormethylthio oder

$R^1$ und $R^2$ gemeinsam mit dem Stickstoffatom, an welches sie gebunden sind, für einen gegebenenfalls ein- bis dreifach, gleich oder verschieden substituierten Heterocyclus der Formel

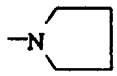 ; 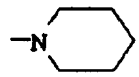 ; 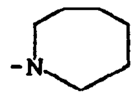 ; 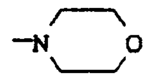 ;

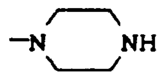 ; 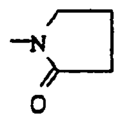 ; 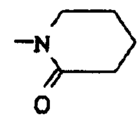 ; 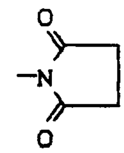 ;

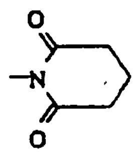 ; 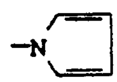 oder 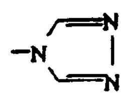

stehen,

wobei als Substituenten infrage kommen: Methyl, Ethyl, n- oder i-Propyl, Chlor oder Trifluormethyl,

$R^3$ für Wasserstoff oder Methyl steht,

$R^4$ für Methyl, Ethyl, n- oder i-Propyl, n-, i-, s- oder t-Butyl, für jeweils geradkettiges oder verzweigtes Pentyl, Hexyl, Heptyl, Octyl, Nonyl, Decyl oder Dodecyl, für Allyl, für jeweils geradkettiges der verzweigtes Butenyl, Pentenyl, Hexenyl, Propargyl, Butinyl, Pentinyl oderHexinyl, für geradkettiges oder verzweigtes Halogenalkyl mit 1 bis 6 Kohlenstoffatomen und 1 bis 9 gleichen oder verschiedenen Halogenatomen, insbesondere Fluor, Chlor oder Brom, für jeweils geradkettiges oder verzweigtes Halogenalkenyl oder Halogenalkinyl mit jeweils 3 bis 5 Kohlenstoffatomen und 1 bis 3 Halogenatomen, insbesondere Fluor oder Chlor, für jeweils geradkettiges oder verzweigtes Cyanalkyl mit 1 bis 6 Kohlenstoffatomen im Alkylteil, Hydroxyalkyl mit 1 bis 6 Kohlenstoffatomen und 1 bis 3 Hydroxygruppen, Alkoxyalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl- und Alkenylteilen oder für jeweils gegebenenfalls ein- bis dreifach, gleich oder verschieden substituiertes Cyclopropyl, Cyclopropylmethyl, Cyclopropylethyl, Cyclopentyl, Cyclohexyl, Cycloheptyl, Cyclooctyl, Cyclohexylmethyl, Cyclohexylethyl, Cyclohexenyl oder Cyclohexenylmethyl steht, wobei als Substituenten jeweils infrage kommen: Fluor, Chlor, Brom, Methyl, Ethyl, n-oder i-Propyl, n-, i-, s-oder t-Butyl, Cyano, Methandiyl, Ethandiyl, Butandiyl oder Butadiendiyl;

außerdem für im Heterocyclylteil gegebenenfalls ein- bis dreifach, gleich oder verschieden substituiertes Heterocyclylmethyl, Heterocyclylpropyl oder Heterocyclylethyl steht, wobei als Heterocyclen jeweils infrage kommen;

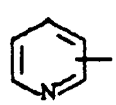 ; 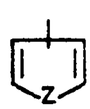 ; 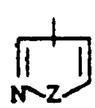 ; 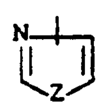 ;

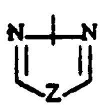 ; 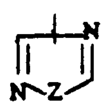 ; 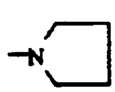 ; 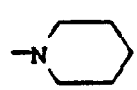 ;

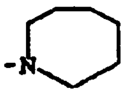 ; 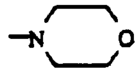 oder 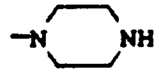

wobei Z jeweils für Sauerstoff oder Schwefel steht und wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio; $R^4$ außerdem für jeweils geradkettiges oder verzweigtes Alkoxy mit 1 bis 6 Kohlenstoffatomen, Alkenyloxy mit 3 bis 6 Kohlenstoffatomen oder Alkinyloxy mit 3 bis 6 Kohlenstoffatomen oder für jeweils gegebenenfalls ein- bis dreifach, gleich oder verschieden substituiertes, gegebenenfalls geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl, Phenylcyanpropyl, Benzyloxy, Phenylethyloxy, Phenoxy, Benzoyl, Phenyl oder Naphthyl steht, wobei als Phenylsubstituenten jeweils infrage kommen: Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Trifuormethylsulfinyl, Trifluormethylsulfonyl, Methylsulfinyl, Methylsulfonyl, Acetyl, Propionyl, Methoxycarbonyl, Ethoxycarbonyl, Cyclohexyl oder Phenoxy; oder

$R^3$ und $R^4$ gemeinsam mit dem Stickstoffatom, an welches sie gebunden sind, für einen gegebenenfalls ein- bis dreifach, gleich oder verschieden substituierten Heterocyclus der Formel

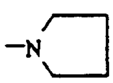 ; 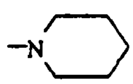 ; 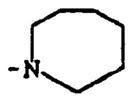 ; 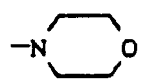 ;

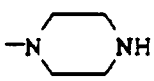 ; 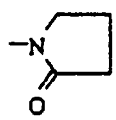 ; 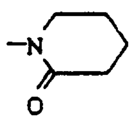 ; 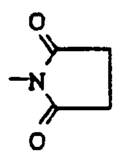 ;

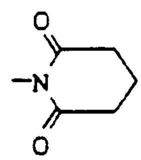 ; 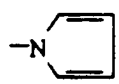 oder 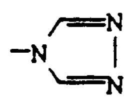

stehen,

wobei als Substituenten infrage kommen: Methyl, Ethyl, n- oder i-Propyl, Chlor oder Trifluormethyl,

$R^5$ für Methyl, Ethyl, Propyl, Isopropyl, n-, i-, s- oder t-Butyl, Cyclopropyl, Cyclobutyl, Cyclopentyl oder Cyclohexyl steht und

X für Sauerstoff oder Schwefel steht.

Ganz besonders bevorzugt sind die Verbindungen der Formel (I), bei welchen

$R^1$ für Methyl, Ethyl, n- oder i-Propyl, n-, i-, s- oder t-Butyl, n- oder i-Pentyl, Cyclopropyl, Cyclopentyl oder Cyclohexyl steht,

$R^2$ für Methyl, Ethyl, n- oder i-Propyl, n-, i-, s- oder t-Butyl, n- oder i-Pentyl steht oder

$R^1$ und $R^2$ gemeinsam mit dem Stickstoffatom, an welches sie gebunden sind, für einen Heterocyclus der Formel

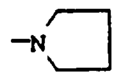 ; 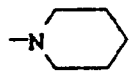 ; 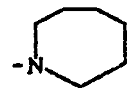 ; 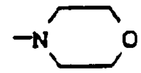

oder

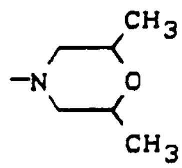

stehen,

$R^3$ für Wasserstoff steht,

$R^4$ für Methyl, Ethyl, n- oder i-Propyl, n-, i-, s- oder t-Butyl, Allyl, Propargyl, jeweils geradkettiges oder verzweigtes Pentyl, Hexyl, Heptyl, Butenyl, Pentenyl, Butinyl, Pentinyl oder Hexinyl, Halogenalkyl mit 1 bis 5 Kohlenstoffatomen und 1 bis 9 gleichen oder verschiedenen Halogenatomen, insbesondere Fluor oder Chlor, jeweils gegebenenfalls einfach, zweifach oder dreifach, gleich oder verschieden durch Methyl, Ethyl, Fluor oder Chlor substituiertes Cyclopropyl, Cyclopentyl oder Cyclohexyl, jeweils gegebenenfalls einfach, zweifach oder dreifach, insbesondere einfach, gleich oder verschieden substituiertes Phenyl, Benzyl oder Phenylethyl steht, wobei als Substituenten Fluor, Chlor, Methyl, Ethyl, Methoxy, Ethoxy, Methylthio, Trifluormethoxy und Trifluormethylthio ausgewählt sind und wobei Benzyl oder Phenylethyl insbesondere unsubstiutiert sind,

$R^5$ für Methyl, Ethyl, n-, oder i-Propyl, insbesondere für Methyl steht und

X für Sauerstoff steht.

Verwendet man beispielsweise 5-Dimethylamino-1-methyl-1,2-dihydro-3H-1,2,4-triazol-3-on und tert-Butyl-isocyanat als Ausgangsstoffe, so läßt sich der Reaktionsablauf des erfindungsgemäßen Verfahrens (a) durch das folgende Formelschema wiedergeben:

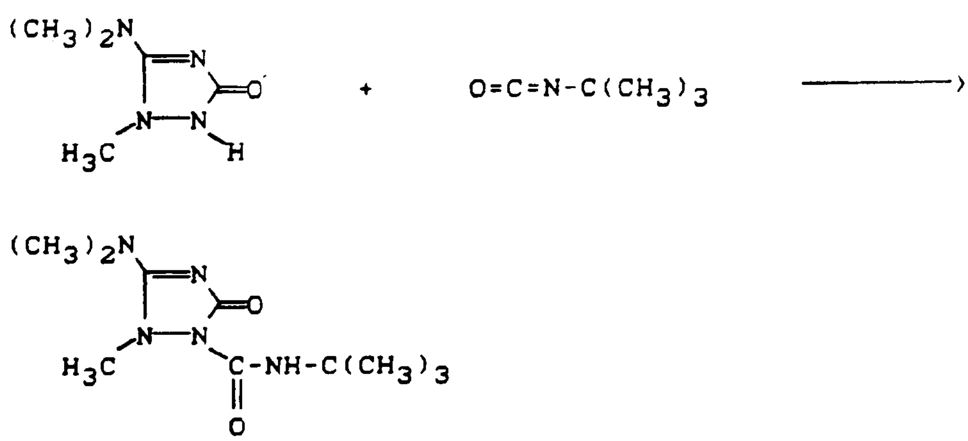

Verwendet man beispielsweise 2-Chlorcarbonyl-1-methyl-5-piperidino-1,2-dihydro-3H-1,2,4-triazol-3-on und Diethylamin als Ausgangsstoffe, so läßt sich der Reaktionsablauf des erfindungsgemäßen Verfahrens (b) durch das folgende Formelschema wiedergeben:

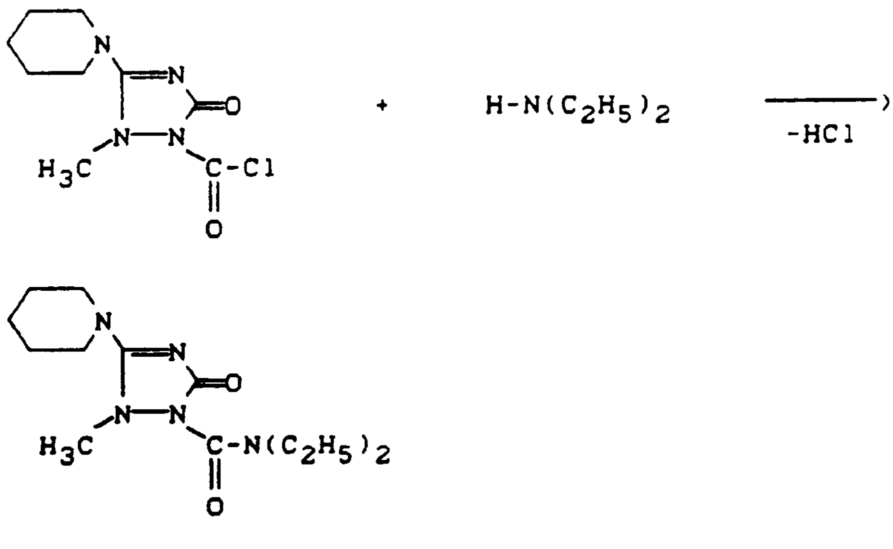


Die Reste ($R^1$, $R^2$, X usw.), die in den Wirkstoffen der Formel (I) definiert sind, haben in den Zwischen- und Vorprodukten für alle Definitionsbereiche ebenfalls die bei den Verbindungen der Formel (I) angegebenen Bedeutungen. Entsprechendes gilt auch für die Reste, die in Vor- und Zwischenprodukten mehrmals genannt sind.

Die Ausgangsstoffe der Formel (II) bzw. die dazu tautomeren Hydroxytriazole sind bekannt und/oder können nach an sich bekannten Verfahren hergestellt werden (vgl. DE-OS 2042660, DE-OS 2330089, DE-OS 2428204, DE-OS 2537973).

Man erhält die Triazolinone der Formel (II) beispielsweise, wenn man Cyanamide der Formel (VI)

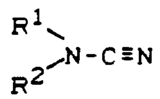 (VI)

in welcher
$R^1$ und $R^2$ die oben angegebene Bedeutung haben,
mit Chlorameisensäureestern der Formel (VII)

Cl-COOR (VII)

in welcher
R für Methyl, Ethyl oder Phenyl, vorzugsweise für Phenyl, steht,
bei Temperaturen zwischen 20°C und 150°C umsetzt und die hierbei gebildeten Chlorformamidin-Derivate der Formel (VIII)

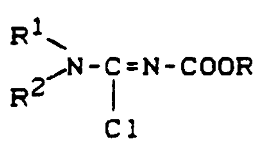 (VIII)

in welcher
R, $R^1$ und $R^2$ die oben angegebene Bedeutung haben,
gegebenenfalls nach Isolierung durch Vakuumdestillation oder nach anderen üblichen Methoden,
mit Alkylhydrazinen der Formel (IX)

$R^5$-NH-$NH_2$ (IX)

in welcher
$R^5$ die oben angegebene Bedeutung hat,
gegebenenfalls in Gegenwart von Verdünnungsmitteln, wie z.B. Methylenchlorid, Dioxan und/oder Diethylether, bei Temperaturen zwischen 0°C und 100°C umsetzt und nach üblichen Methoden aufarbeitet (vgl. die Herstellungsbeispiele).

Die Ausgangsstoffe der Formel (III) sind bekannte organische Synthesechemikalien.

Die Ausgangsstoffe der Formel (IV) sind noch nicht aus der Literatur bekannt.

Man erhält die neuen Chlorcarbonyl-triazolinone der Formel (IV), wenn man Triazolinone der Formel (II)

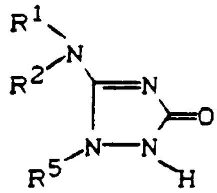

(II)

in welcher

$R^1$, $R^2$ und $R^5$ die oben angegebene Bedeutung haben,

mit Phosgen, gegebenenfalls in Gegenwart eines Säurebindemittels, wie z.B. Triethylamin, und gegebenenfalls in Gegenwart eines Verdünnungsmittels, wie z.B. Toluol oder Acetonitril, bei Temperaturen zwischen 0°C und 150°C umsetzt.

Die Ausgangsstoffe der Formel (V) sind bekannte organische Synthesechemikalien.

Das erfindungsgemäße Verfahren (a) zur Herstellung der neuen substituierten Triazolinone der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Das erfindungsgemäße Verfahren (a) kann gegebenenfalls in Gegenwart eines basischen Reaktionshilfsmittels durchgeführt werden. Als solche kommen alle üblichen anorganischen und organischen Basen infrage. Vorzugsweise verwendet man tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Der Zusatz solcher Katalysatoren ist jedoch nicht zwingend erforderlich.

Die Reaktionstemperaturen können bei der Durchführung des erfindungsgemäßen Verfahrens (a) in einem größeren Bereich variiert werden. Im allgemeinen arbeitet man bei Temperaturen zwischen 0 °C und + 150 °C, vorzugsweise bei Temperaturen zwischen + 40 °C und + 120 °C.

Das erfindungsgemäße Verfahren (a) wird üblicherweise unter Normaldruck durchgeführt. Es ist jedoch auch möglich, insbesondere bei gasförmigen Ausgangsverbindungen, unter erhöhtem Druck zu arbeiten.

Zur Durchführung des erfindungsgemäßen Verfahrens (a) setzt man pro Mol Triazolinon der Formel (II) im allgemeinen 1 bis 5 Mol, vorzugsweise 1 bis 2,5 Mol an Iso(thio)cyanat der Formel (III) und gegebenenfalls 1 bis 2,5 Mol an Reaktionshilfsmittel ein. Die Reaktionsdurchführung, Aufarbeitung und Isolierung der Reaktionsprodukte erfolgt in Analogie zu allgemein bekannten Verfahren (vgl. die Herstellungsbeispiele).

Als Verdünnungsmittel zur Durchführung des erfindungsgemäßen Verfahrens (b) kommen ebenfalls inerte organische Lösungsmittel infrage. Vorzugsweise verwendet man die bei Verfahren (a) genannten Verdünnungsmittel.

Das erfindungsgemäße Verfahren (b) wird gegebenenfalls in Gegenwart eines geeigneten Säurebindemittels durchgeführt. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Es ist auch möglich das als Reaktionspartner verwendete Amin der Formel (V) in entsprechendem Überschuß gleichzeitig als Säurebindemittel einzusetzen.

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und + 150 °C, vorzugsweise bei Temperaturen zwischen + 10 °C und + 80 °C.

Das erfindungsgemäße Verfahren (b) wird üblicherweise unter Normaldruck durchgeführt. Es ist jedoch auch möglich unter erhöhtem Druck zu arbeiten.

Zur Durchführung des erfindungsgemäßen Verfahrens (b) setzt man pro Mol Chlorcarbonyl-triazolinon der

Formel (IV) im allgemeinen 1 bis 5 Mol, vorzugsweise 1 bis 2,5 Mol Amin der Formel (V) und gegebenenfalls 1 bis 2,5 Mol Säurebindemittel ein. Die Reaktionsdurchführung, Aufarbeitung und Isolierung der Reaktionsprodukte erfolgt in Analogie zu allgemein bekannten Verfahren (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen der Formel (I) eignen sich insbesondere zur selektiven Bekämpfung von dikotylen Unkräutern in monokotylen Kulturen im Vorauflauf- und im Nachauflauf-Verfahren, vor allem im Nachauflauf-Verfahren.

In gewissem Umfang zeigen die Verbindungen der Formel (I) auch Defoliant-Wirkung in Baumwolle, Wirkung gegen Pyricularia oryzae und/oder auch blattinsektizide und akarizide Wirkung.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische

pulvrige, körnige oder latexförmige Polymere verwendet werden, wie Gummi arabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); Chloressigsäure-N-(methoxymethyl)-2,6-diethylanilid (ALACHLOR); 2-Chlor-4-ethylamino-6-isopropylamino-1,3,5-triazin (ATRAZIN); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); 2-Chlor-4-ethylamino-6-(3-cyanopropylamino)-1,3,5-triazin (CYANAZIN); 2-[4-(2,4-Dichlorphenoxy)-phenoxy]-propionsäure, deren Methyl-oder deren Ethylester (DICLOFOP); N,N-Di-n-propyl-thiocarbamidsäure-S-ethylester (EPTAME); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl-oder deren Ethylester (FENOXAPROP); [(4-Amino-3,5-dichlor-6-fluor-2-pyridinyl)-oxy]-essigsäure bzw. deren 1-Methylheptylester (FLUROXYPYR); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N'-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 0-(6-Chlor-3-phenyl-pyridazin-4-yl)-S-octyl-thiocarbonat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE); einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele:

Beispiel 1

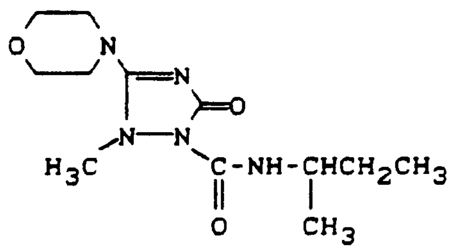

(Verfahren (a))

2,5 g (25 mMol) sec-Butyl-isocyanat werden zu einer Mischung aus 4,6 g (25 mMol) 1-Methyl-5-morpholino-1,2-dihydro-3H-1,2,4-triazol-3-on und 150 ml Acetonitril gegeben und nach Zugabe von 2 Tropfen 1,8-Diazabicyclo-[5.4.0]-undec-7-en (DBU) wird das Reaktionsgemisch 2 Stunden bei Rückflußtemperatur gerührt. Nach dem Abkühlen wird abgesaugt und das feste Produkt aus Hexan/Essigsäureethylester (Vol. 1:1) umkristallisiert.

Man erhält 4,4 g (62% der Theorie) 2-(sec-Butyl-aminocarbonyl)-1-methyl-5-morpholino-1,2-dihydro-3H-1,2,4-triazol-3-on vom Schmelzpunkt 147°C.

$^1$H-NMR ($CDCl_3$, δ, ppm): 1,15-1,25; 3,35; 7,95-8,00.

Beispiel 2

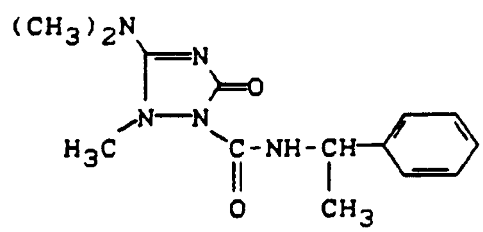

(Verfahren (b))

Zu einer Mischung aus 7,8 g (38 mMol) 2-Chlorcarbonyl-5-dimethylamino-1-methyl-1,2-dihydro-3H-1,2,4-triazol-3-on und 150 ml Acetonitril werden unter Rühren 9,2 g (76 mMol) 1-Phenyl-ethylamin so zugetropft, daß die Reaktionstemperatur 40°C nicht übersteigt. Dann wird das Reaktionsgemisch 2 Stunden bei 20°C gerührt und anschließend filtriert. Das Filtrat wird im Wasserstrahlvakuum eingeengt, der Rückstand mit Methylenchlorid/Wasser geschüttelt, die organische Phase mit Natriumsulfat getrocknet und filtriert. Vom Filtrat wird das Lösungsmittel im Wasserstrahlvakuum abdestilliert und der feste Rückstand aus Hexan/Essigsäureethylester (Vol. 4:1) umkristallisiert.

Man erhält 3,83 g (35% der Theorie) 5-Dimethylamino-1-methyl-2-(1-phenyl-ethylamino-carbonyl)-1,2-dihydro-3H-1,2,4-triazol-3-on vom Schmelzpunkt 103°C.

$^1$H-NMR ($CDCl_3$, δ, ppm): 1,50-1,55; 3,15; 3,30; 7,20-7,35; 8,50-8,60.

Analog zu den Beispielen 1 und 2 und entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

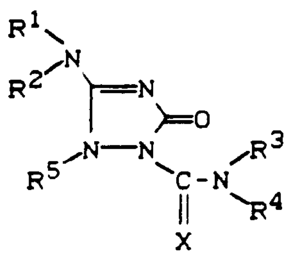

(I)

Tabelle 1: Beispiele für die Verbindungen der Formel (I)

| Bsp.-Nr. | 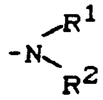 | $R^3$ | $R^4$ | $R^5$ | X | Schmelzpunkt (°C) bzw. $^1H$-NMR |
|---|---|---|---|---|---|---|
| 3 | $-N(CH_3)_2$ | H | 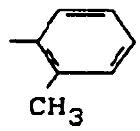 | $CH_3$ | O | 131 |
| 4 | $-N(CH_3)_2$ | H | $-(CH_2)_3-CH_3$ | $CH_3$ | O | $^1H$-NMR*): 0,90-0,95; 3,15;3,35 |
| 5 | $-N(CH_3)_2$ | H | $-CH_2C(CH_3)_3$ | $CH_3$ | O | 77 |
| 6 | $-N(CH_3)_2$ | H | 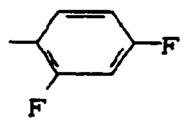 | $CH_3$ | O | 207 |
| 7 | $-N(C_2H_5)_2$ | H | $-CH(CH_3)_2$ | $CH_3$ | O | $^1H$-NMR*): 1,20-1,25; 1,25-1,30; 3,30; 3,45-3,55; 3,95-4,05 |
| 8 | 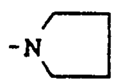 | H | $-CH(CH_3)_2$ | $CH_3$ | O | 133 |
| 9 | $-N(CH_3)_2$ | H | $-C(CH_3)_2CH_2Cl$ | $CH_3$ | O | 135 |
| 10 | $-N(CH_3)_2$ | H | $-CHCH_2CH_3$<br>\|<br>$CH_3$ | $CH_3$ | O | $^1H$-NMR*): 1,15-1,20; 1,50-1,60; 3,15;3,35; 3,75-3,85 |
| 11 | $-N(CH_3)_2$ | H | $-CH(CH_3)_2$ | $CH_3$ | O | 63 |
| 12 | $-N(CH_3)_2$ | H | 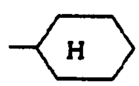 | $CH_3$ | O | 82 |
| 13 | $N(CH_3)_2$ | H | $-C(CH_3)_2CH_2F$ | $CH_3$ | O | 110 |

Tabelle 1 - Fortsetzung

| Bsp.-Nr. | $-N(R^1)(R^2)$ | $R^3$ | $R^4$ | $R^5$ | X | Schmelzpunkt (°C) bzw. $^1H$-NMR |
|---|---|---|---|---|---|---|
| 14 | $-N(CH_3)_2$ | H | $-C(CH_3)_3$ | $CH_3$ | O | 135 |
| 15 | $-N(CH_3)_2$ | H | 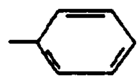 | $CH_3$ | O | 158 |
| 16 | $-N(C_2H_5)_2$ | H | $-C(CH_3)_3$ | $CH_3$ | O | 108 |
| 17 | $-N(C_2H_5)_2$ | H | $-C(CH_3)_2CH_2Cl$ | $CH_3$ | O | 82 |
| 18 | $-N(CH_3)_2$ | H | 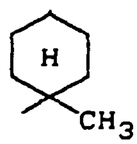 | $CH_3$ | O | 140 |
| 19 | $-N(CH_3)_2$ | H | $-CH_2CH=CH_2$ | $CH_3$ | O | $^1H$-NMR*): 3,15;3,35; 3,90-3,95; 5,70-5,95; 8,25-8,30 |
| 20 | $-N(CH_3)_2$ | H | $-CH_2CH_2CH_3$ | $CH_3$ | O | $^1H$-NMR*): 0,90-1,00; 1,55-1,65; 3,15;3,35 |
| 21 | $-N(CH_3)_2$ | H | $C_2H_5$ | $CH_3$ | O | 81 |
| 22 | $-N(CH_3)_2$ | H | $-CH(C_2H_5)_2$ | $CH_3$ | O | $^1H$-NMR*): 0,90-1,00; 1,40-1,65; 3,15;3,35 |
| 23 | $-N(CH_3)_2$ | H | $-CH_2CH_2Cl$ | $CH_3$ | O | $^1H$-NMR*): 3,15;3,35; 3,65 |
| 24 | $-N(CH_3)_2$ | H | $-\underset{\mid \atop CH_3}{C}HCH(CH_3)_2$ | $CH_3$ | O | $^1H$-NMR*): 0,90-0,95; 1,15-1,20; 1,70-1,85; 3,15;3,35 |

Tabelle 1 - Fortsetzung

| Bsp.-Nr. | $-N(R^1)(R^2)$ | $R^3$ | $R^4$ | $R^5$ | X | Schmelzpunkt (°C) bzw. ¹H-NMR |
|---|---|---|---|---|---|---|
| 25 | $-N(CH_3)_2$ | H | 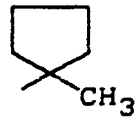 | $CH_3$ | O | 127 |
| 26 | $-N(C_2H_5)_2$ | H | 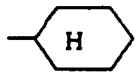 | $CH_3$ | O | 105 |
| 27 | $-N(C_2H_5)_2$ | H | 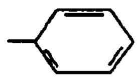 | $CH_3$ | O | 107 |
| 28 | $-N(CH_3)_2$ | H | $-C(CH_2Cl)_2$<br>\|<br>$CH_3$ | $CH_3$ | O | 99 |
| 29 | $-N(CH_3)_2$ | H | $-C(CH_3)_2CHCl_2$ | $CH_3$ | O | 93 |
| 30 | $-N(CH_3)_2$ | H | 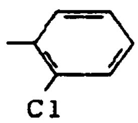 | $CH_3$ | O | 109 |
| 31 | $-N(CH_3)_2$ | H | 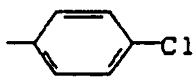 | $CH_3$ | O | 172 |
| 32 | $-N(CH_3)_2$ | H | 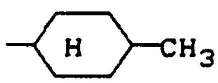 | $CH_3$ | O | 143 |
| 33 | $-N(CH_3)_2$ | H | 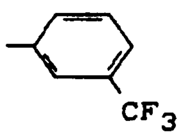 | $CH_3$ | O | 177 |
| 34 | $-N(CH_3)_2$ | H | 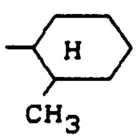 | $CH_3$ | O | 109 |

Tabelle 1 - Fortsetzung

| Bsp.-Nr. | 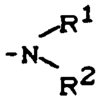 | $R^3$ | $R^4$ | $R^5$ | X | Schmelzpunkt (°C) bzw. $^1$H-NMR |
|---|---|---|---|---|---|---|
| 35 | $-N(CH_3)_2$ | H | $-CH_2$ 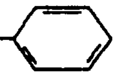 | $CH_3$ | O | 76 |
| 36 | 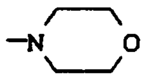 | H | $-C(CH_3)_3$ | $CH_3$ | O | 148 |
| 37 | 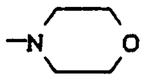 | H | 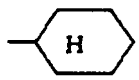 | $CH_3$ | O | 165 |
| 38 | 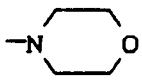 | H | 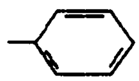 | $CH_3$ | O | 225 |
| 39 | $-N[CH(CH_3)_2]_2$ | H | $-CH(CH_3)CH_2CH_3$ | $CH_3$ | O | 124 |
| 40 | $-N[CH(CH_3)_2]_2$ | H | $-C(CH_3)_3$ | $CH_3$ | O | 140 |
| 41 | $-N[CH(CH_3)_2]_2$ | H | 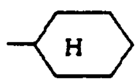 | $CH_3$ | O | 166 |
| 42 | $-N[CH(CH_3)_2]_2$ | H | 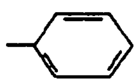 | $CH_3$ | O | 172 |
| 43 | 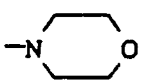 | $CH_3$ | $CH_3$ | $CH_3$ | O | 138 |
| 44 | 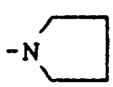 | H | 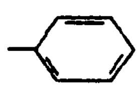 | $CH_3$ | O | 191 |
| 45 | 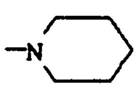 | H | 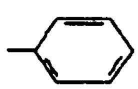 | $CH_3$ | O | 177 |

<u>Tabelle 1</u> - Fortsetzung

| Bsp.-Nr. | 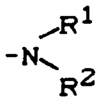 | $R^3$ | $R^4$ | $R^5$ | X | Schmelzpunkt ($^\circ C$) bzw. $^1H$-NMR |
|---|---|---|---|---|---|---|
| 46 | 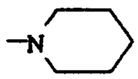 | H | $-C(CH_3)_3$ | $CH_3$ | O | 121 |
| 47 | 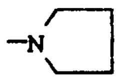 | H | 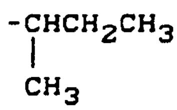 | $CH_3$ | O | $^1H$-NMR*): 0,90-0,95; 1,18-1,22; 2,00-2,05; 3,35; 3,60-3,65 |
| 48 | 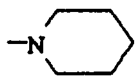 | H | 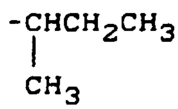 | $CH_3$ | O | $^1H$-NMR*): 1,18-1,22; 1,15-1,20; 3,20; 3,55-3,60; 3,75-3,90 |
| 49 | 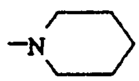 | H | $-CH(CH_3)_2$ | $CH_3$ | O | 112 |
| 50 | 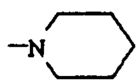 | H | 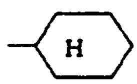 | $CH_3$ | O | 120 |
| 51 | 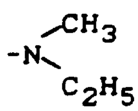 | H | 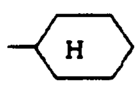 | $CH_3$ | O | 84 |
| 52 | 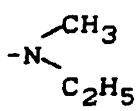 | H | $-C(CH_3)_3$ | $CH_3$ | O | 83 |
| 53 | 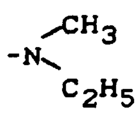 | H | 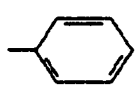 | $CH_3$ | O | 126 |

Tabelle 1 - Fortsetzung

| Bsp.-Nr. | 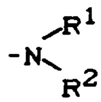 | $R^3$ | $R^4$ | $R^5$ | X | Schmelzpunkt (°C) bzw. $^1$H-NMR |
|---|---|---|---|---|---|---|
| 54 | 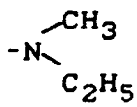 | H | $-CH(CH_3)_2$ | $CH_3$ | O | $^1$H-NMR*): 1,20-1,23; 1,25-1,30; 3,1;3,3; 3,50-3,60; 3,95-4,05 |
| 55 | 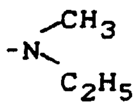 | H | $-C(CH_3)_2CH_2F$ | $CH_3$ | O | 77 |
| 56 | $-N(CH_3)_2$ | H | $-C(CH_3)_2C{\equiv}CH$ | $CH_3$ | O | 163 |
| 57 | 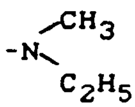 | H | 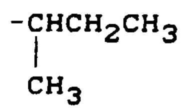 | $CH_3$ | O | $^1$H-NMR*): 1,18-1,22; 1,25-1,30; 1,50-1,60; 3,15;3,35 |
| 58 | $-N(CH_3)_2$ | H | 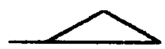 | $CH_3$ | O | 115 |
| 59 | $-N(CH_3)_2$ | H | 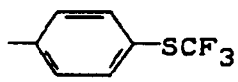 | $CH_3$ | O | |
| 60 | $-N(CH_3)_2$ | H | 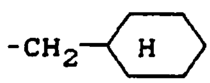 | $CH_3$ | O | $^1$H-NMR*): 0,90-1,80; 3,15;3,35 |
| 61 | 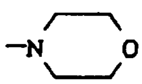 | H | 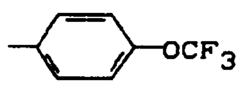 | $CH_3$ | O | 183 |
| 62 | $-N[CH(CH_3)_2]_2$ | H | 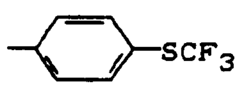 | $CH_3$ | O | 192 |

<u>Tabelle 1</u> - Fortsetzung

| Bsp.-Nr. | 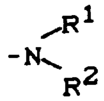 | $R^3$ | $R^4$ | $R^5$ | X | Schmelzpunkt (°C) bzw. $^1H$-NMR |
|---|---|---|---|---|---|---|
| 63 | 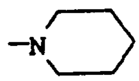 | H | 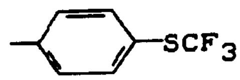 | $CH_3$ | O | 192 |
| 64 | 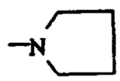 | H | 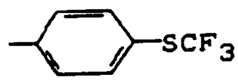 | $CH_3$ | O | 182 |
| 65 | 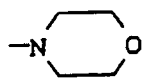 | H | 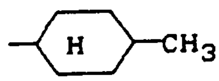 | $CH_3$ | O | 169 |
| 66 | 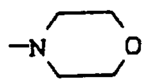 | H | 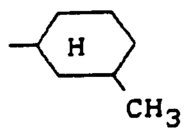 | $CH_3$ | O | 150 |
| 67 | 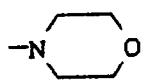 | H | 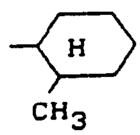 | $CH_3$ | O | 159 |
| 68 | $-N(CH_3)_2$ | H | $CH_3$ | $CH_3$ | S | 103 |
| 69 | 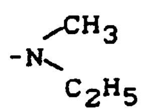 | H | $CH_3$ | $CH_3$ | S | (amorph) |
| 70 | 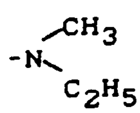 | H | $C_2H_5$ | $CH_3$ | S | 83 |
| 71 | 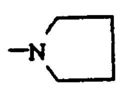 | H | $-C(CH_3)_3$ | $CH_3$ | O | 132 |
| 72 | 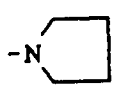 | H | 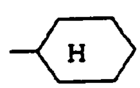 | $CH_3$ | O | 94 |

Tabelle 1 - Fortsetzung

| Bsp.-Nr. | 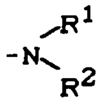 | $R^3$ | $R^4$ | $R^5$ | X | Schmelzpunkt (°C) bzw. $^1$H-NMR |
|---|---|---|---|---|---|---|
| 73 | 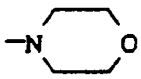 | H | $-CH(CH_3)_2$ | $CH_3$ | O | 172 |
| 74 | 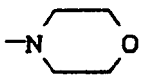 | H | $-C(CH_3)_2CH_2F$ | $CH_3$ | O | 159 |
| 75 | 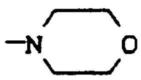 | H | $-CH_2C(CH_3)_3$ | $CH_3$ | O | 152 |
| 76 | 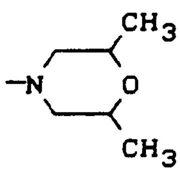 | H | 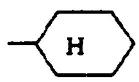 | $CH_3$ | O | 132 |
| 77 | $-N(CH_3)_2$ | H | 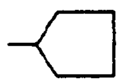 | $CH_3$ | O | 62 |
| 78 | 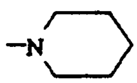 | H | 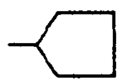 | $CH_3$ | O | 99 |
| 79 | 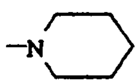 | H | 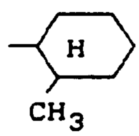 | $CH_3$ | O | 125 |
| 80 | 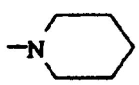 | $CH_3$ | $CH_3$ | $CH_3$ | O | 130 |
| 81 | 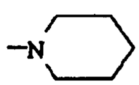 | H | 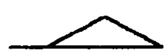 | $CH_3$ | S | 136 |

Tabelle 1 - Fortsetzung

| Bsp.-Nr. | $-N\langle^{R^1}_{R^2}$ | $R^3$ | $R^4$ | $R^5$ | X | Schmelzpunkt (°C) bzw. $^1H$-NMR |
|---|---|---|---|---|---|---|
| 82 | 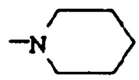 | H | $CH_3$ | $CH_3$ | S | 114 |
| 83 | 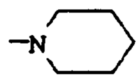 | H | $-CH(C_2H_5)_2$ | $CH_3$ | O | (amorph) |
| 84 | 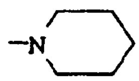 | H | $-\underset{CH_3}{\underset{\mid}{C}}HCH(CH_3)_2$ | $CH_3$ | O | (amorph) |
| 85 | 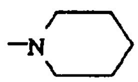 | H | $-CH_2C(CH_3)_3$ | $CH_3$ | O | |
| 86 | 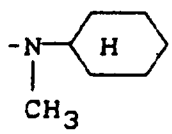 | H | 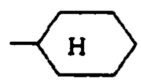 | $CH_3$ | O | 117 |
| 87 | 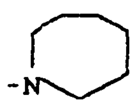 | H | 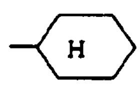 | $CH_3$ | O | 113 |
| 88 | 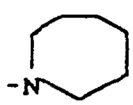 | H | $-CH(CH_3)_2$ | $CH_3$ | O | 88 |
| 89 | 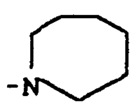 | H | $-\underset{CH_3}{\underset{\mid}{C}}HCH_2CH_3$ | $CH_3$ | O | (amorph) |
| 90 | $-N(CH_3)_2$ | H | $-CH_2CH(CH_3)_2$ | $CH_3$ | O | (amorph) |
| 91 | 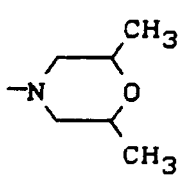 | H | $-CH(CH_3)_2$ | $CH_3$ | O | $^1H$-NMR*): 1,15-1,25; 3,35; 3,95-4,05 |

Tabelle 1 - Fortsetzung

| Bsp.-Nr. | 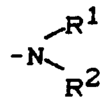 | $R^3$ | $R^4$ | $R^5$ | X | Schmelzpunkt ($^{\circ}$C) bzw. $^{1}$H-NMR |
|---|---|---|---|---|---|---|
| 92 | $-N(CH_3)_2$ | H | 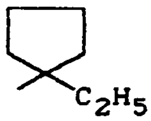 | $CH_3$ | O | $^{1}$H-NMR*): 0,85-0,90; 1,80-1,90; 1,55-1,80; 3,15;3,35 |
| 93 | $-N(CH_3)_2$ | H | 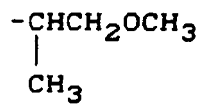 | $CH_3$ | O | $^{1}$H-NMR*): 1,20-1,25; 3,15;3,35; 4,05-4,15 |
| 94 | 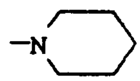 | H | 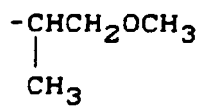 | $CH_3$ | O | $^{1}$H-NMR*): 1,20-1,25; 3,30; 3,35-3,40; 4,05-4,15 |
| 95 | $-N(CH_3)_2$ | H | 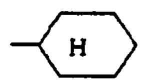 | $-CH(CH_3)_2$ | O | 118$^{\circ}$ C |
| 96 | $-N(CH_3)_2$ | H | $-CH(CH_3)_2$ | $-CH(CH_3)_2$ | O | 83$^{\circ}$ C |
| 97 | 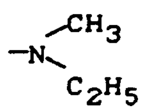 | H | 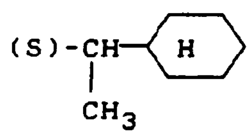 | $CH_3$ | O | $^{1}$H-NMR*): 1,14-1,17; 3,10;3,32 3,70-3,82; 8,05-8,10 |
| 98 | 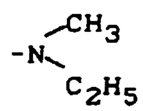 | H | 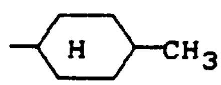 | $CH_3$ | O | 97$^{\circ}$ C |
| 99 | 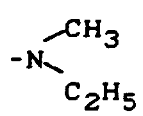 | H | 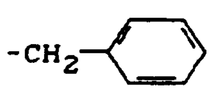 | $CH_3$ | O | 97$^{\circ}$ C |

Tabelle 1 - Fortsetzung

| Bsp.-Nr. | 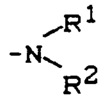 | $R^3$ | $R^4$ | $R^5$ | X | Schmelzpunkt (°C) bzw. $^1$H-NMR |
|---|---|---|---|---|---|---|
| 100 | 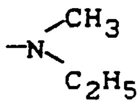 | H | 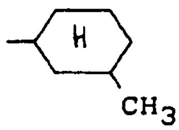 | $CH_3$ | O | $^1$H-NMR*): 0,90-0,92; 3,10;3,30 3,55-3,63; |
| 101 | 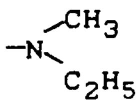 | H | 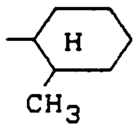 | $CH_3$ | O | $^1$H-NMR*): 0,90-0,96; 3,10;3,30 3,50-3,56; |
| 102 | 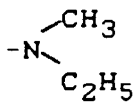 | H | 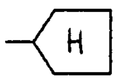 | $CH_3$ | O | 54° C |
| 103 | 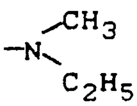 | H | 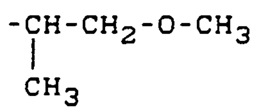 | $CH_3$ | O | $^1$H-NMR*): 1,20-1,30; 3,10;3,32 3,37; 3,49-3,56 |
| 104 | 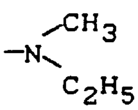 | H | 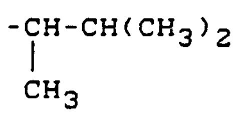 | $CH_3$ | O | $^1$H-NMR*): 0,90-0,95; 1,14-1,16 1,25-1,30; 3,50-3,55 |
| 105 | 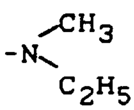 | H | $-CH_2-C(CH_3)_3$ | $CH_3$ | O | $^1$H-NMR*) 0,95;1,25-1,30;3,12; 3,32;3,50-3,60 |
| 106 | 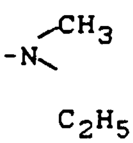 | H | $-CH_2-CH_2-CH_3$ | $CH_3$ | O | $^1$H-NMR*) 0,92-0,98; 3,12; 3,32 3,50-3,60 |

Tabelle 1 - Fortsetzung

| Bsp.-Nr. | 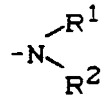 | $R^3$ | $R^4$ | $R^5$ | X | Schmelzpunkt (°C) bzw. $^1H$-NMR |
|---|---|---|---|---|---|---|
| 107 | 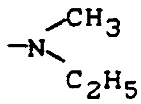 | H | 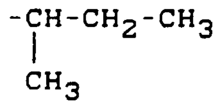 | $CH_3$ | O | $^1H$-NMR*): 1,25-1,30; 1,32-1,35 3,05-3,10; 3,60-3,65 |
| 108 | 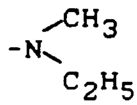 | H | 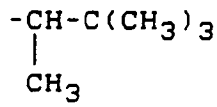 | $CH_3$ | O | $^1H$-NMR*): 0,95; 1,12-1,15; 1,25-1,30; 3,12; 3,32; 3,70-3,80 |
| 109 | 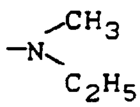 | H | $-(CH_2)_3-CH_3$ | $CH_3$ | O | $^1H$-NMR*): 0,90-0,96; 1,33-1,60; 3,12; 3,25-3,30; 3,32 |
| 110 | 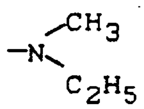 | H | 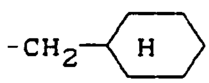 | $CH_3$ | O | $^1H$-NMR*) 1,25-1,30; 3,12; 3,13-3,15; 3,32; 3,50-3,56 |
| 111 | $-N(CH_3)_2$ | H | 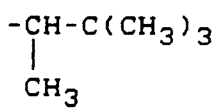 | $CH_3$ | O | 115° C |
| 112 | $-N(CH_3)_2$ | H | 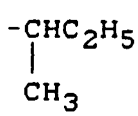 | $CH(CH_3)_2$ | O | 50° C |
| 113 | $-N(CH_3)_2$ | H | 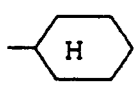 | $CH(CH_3)_2$ | O | 118° C |
| 114 | $-N(CH_3)_2$ | H | $-CH(CH_3)_2$ | $CH(CH_3)_2$ | O | 83° C |
| 115 | $-N(CH_3)_2$ | H | $-C(CH_3)_2CH_2Cl$ | $CH(CH_3)_2$ | O | 81° C |

Tabelle 1 - Fortsetzung

| Bsp.-Nr. | 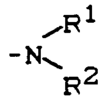 | $R^3$ | $R^4$ | $R^5$ | X | Schmelzpunkt (°C) bzw. $^1H$-NMR |
|---|---|---|---|---|---|---|
| 116 | $-N(CH_3)_2$ | H | $-\underset{\displaystyle CH_3}{\underset{\vert}{C}}H-C_2H_5$ | 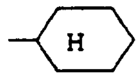 | O | 86° C |
| 117 | $-N(CH_3)_2$ | H | $-CH_2-CH(CH_3)_2$ | $C_2H_5$ | O | $^1H$-NMR*) 0,95-0,98; 0,99-1,05; 1,78-1,90; 3,12-3,15; 3,18; 3,85-3,92 |
| 118 | $-N(CH_3)_2$ | H | 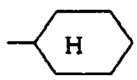 | $C_2H_5$ | O | $^1H$-NMR*) 0,90-1,05; 1,18-1,22; 1,50-1,60; 3,18; 3,80-3,90 |
| 119 | $-N(CH_3)_2$ | H | $-\underset{\displaystyle CH_3}{\underset{\vert}{C}}HC_2H_5$ | $C_2H_5$ | O | (amorph) |
| 120 | $-N(CH_3)_2$ | H | $-CH(CH_3)_2$ | $C_2H_5$ | O | 52° C |
| 121 | $-N(CH_3)_2$ | H | 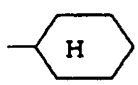 | 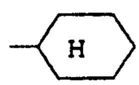 | O | 123° C |
| 122 | $-N(CH_3)_2$ | H | $-CH(CH_3)_2$ | 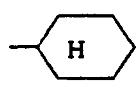 | O | 115° C |

Tabelle 1 - Fortsetzung

| Bsp.-Nr. | 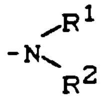 | $R^3$ | $R^4$ | $R^5$ | X | Schmelzpunkt (°C) bzw. $^1H$-NMR |
|---|---|---|---|---|---|---|
| 123 | 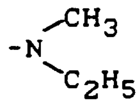 | H | 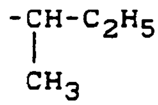 | $CH(CH_3)_2$ | O | $^1H$-NMR*): 3,12; 3,50-3,60; 3,75-3,85; 7,85-7,90 |
| 124 | 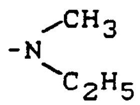 | H | $-CH(CH_3)_2$ | $CH(CH_3)_2$ | O | $^1H$-NMR*): 1,20-1,25; 3,12; 3,50-3,60; 3,75-3,85; 3,95-4,05; 7,89-7,92 |
| 125 | 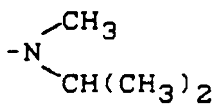 | H | 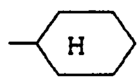 | $CH_3$ | O | 75° C |
| 126 | 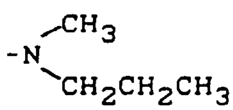 | H | 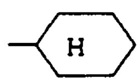 | $CH_3$ | O | $^1H$-NMR*): 3,15; 3,30 3,40-3,50; 3,60-3,75; |
| 127 | 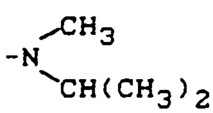 | H | $-CH (CH_3)_2$ | $CH_3$ | O | 155° C |
| 128 | 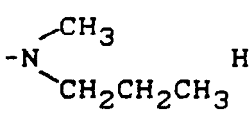 | H | 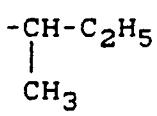 | $CH_3$ | O | $^1H$-NMR*): 1,40-1,45; 3,12; 3,30 3,40-3,50; 3,75-3,90 |

Tabelle 1 - Fortsetzung

| Bsp.-Nr. | 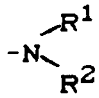 | $R^3$ | $R^4$ | $R^5$ | X | Schmelzpunkt (°C) bzw. $^1$H-NMR |
|---|---|---|---|---|---|---|
| 129 | 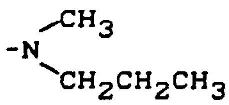 | H | $-CH(CH_3)_2$ | $CH_3$ | O | 62° C |
| 130 | 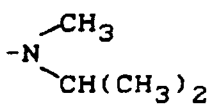 | H | 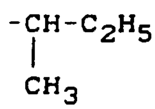 | $CH_3$ | O | 63° C |
| 131 | 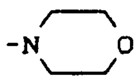 | H | 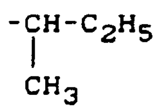 | $C_2H_5$ | O | 72° C |
| 132 | $-N(CH_3)_2$ | H | $-CH_2CH(CH_3)_2$ | 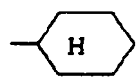 | O | $^1$H-NMR*): 0,90-0,95; 1,10-1,90; 3,08-3,12; 3,15 |
| 133 | $-N(CH_3)_2$ | H | 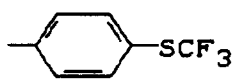 | 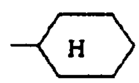 | O | 115° C |
| 134 | 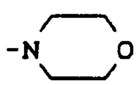 | H | 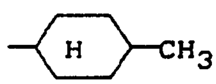 | $C_2H_5$ | O | 108° C |
| 135 | 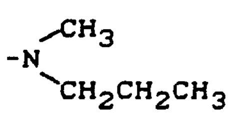 | H | $-CH_2C(CH_3)_3$ | $CH_3$ | O | (amorph) |

Tabelle 1 - Fortsetzung

| Bsp.-Nr. | 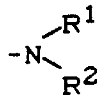 | $R^3$ | $R^4$ | $R^5$ | X | Schmelzpunkt ($^0$C) bzw. $^1$H-NMR |
|---|---|---|---|---|---|---|
| 136 | 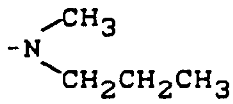 | H | $-CH(C_2H_5)_2$ | $CH_3$ | O | (amorph) |
| 137 | 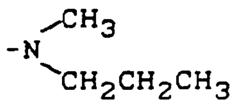 | H | 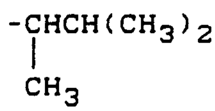 | $CH_3$ | O | (amorph) |
| 138 | 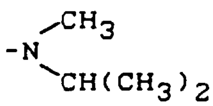 | H | $-CH_2C(CH_3)_3$ | $CH_3$ | O | 100° C |
| 139 | 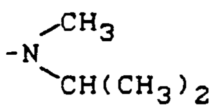 | H | $-CH(C_2H_5)_2$ | $CH_3$ | O | 95° C |
| 140 | 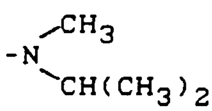 | H | 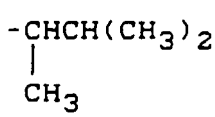 | $CH_3$ | O | 85° C |
| 141 | 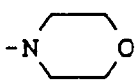 | H | 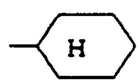 | $CH(CH_3)_2$ | O | 125° C |
| 142 | 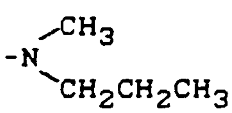 | H | 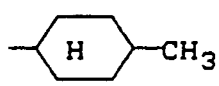 | $CH_3$ | | (amorph) |
| 143 | 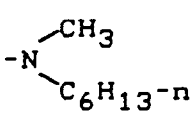 | H | 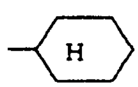 | $CH_3$ | | (amorph) |

Tabelle 1 - Fortsetzung

| Bsp.-Nr. | 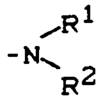 | $R^3$ | $R^4$ | $R^5$ | X | Schmelzpunkt (°C) bzw. $^1$H-NMR |
|---|---|---|---|---|---|---|
| 144 | 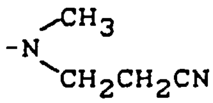 | H | 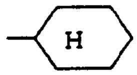 | $CH_3$ | | (amorph) |
| 145 | 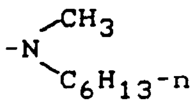 | H | 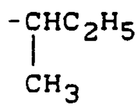 | $CH_3$ | O | (amorph) |
| 146 | 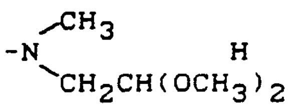 | H | 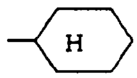 | $CH_3$ | O | (amorph) |
| 147 | 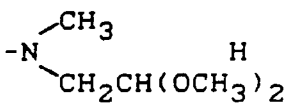 | H | 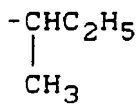 | $CH_3$ | O | (amorph) |
| 148 | 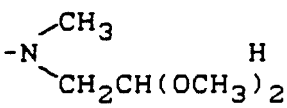 | H | $-CH(CH_3)_2$ | $CH_3$ | O | (amorph) |
| 149 | 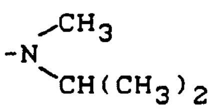 | H | 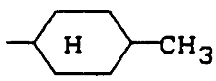 | $CH_3$ | O | (amorph) |
| 150 | 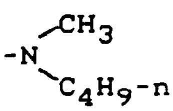 | H | 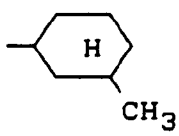 | $CH_3$ | O | (amorph) |

<u>Tabelle 1</u> - Fortsetzung

| Bsp.-Nr. | 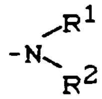 | $R^3$ | $R^4$ | $R^5$ | X | Schmelzpunkt (°C) bzw. $^1$H-NMR |
|---|---|---|---|---|---|---|
| 151 | 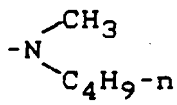 | H | 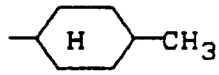 | $CH_3$ | O | (amorph) |
| 152 | 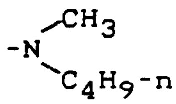 | H | 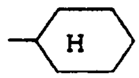 | $CH_3$ | O | (amorph) |
| 153 | 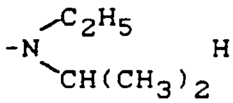 | H | 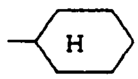 | $CH_3$ | O | 116° C |
| 154 | 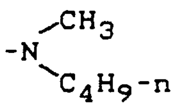 | H | 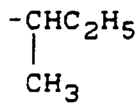 | $CH_3$ | O | (amorph) |
| 155 | 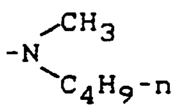 | H | $-CH(CH_3)_2$ | $CH_3$ | O | (amorph) |
| 156 | 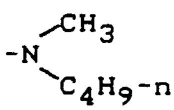 | H | 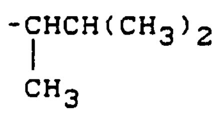 | $CH_3$ | O | (amorph) |
| 157 | 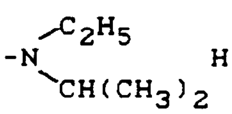 | H | 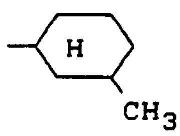 | $CH_3$ | O | (amorph) |

Tabelle 1 - Fortsetzung

| Bsp.-Nr. | 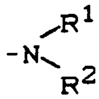 | $R^3$ | $R^4$ | $R^5$ | X | Schmelzpunkt (°C) bzw. ¹H-NMR |
|---|---|---|---|---|---|---|
| 158 | 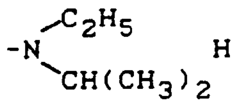 | H | 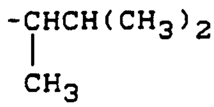 | $CH_3$ | O | (amorph) |
| 159 | 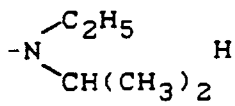 | H | $-CH(CH_3)_2$ | $CH_3$ | O | 80° C |
| 160 | 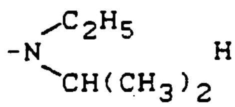 | H | $-CH(C_2H_5)_2$ | $CH_3$ | O | (amorph) |
| 161 | 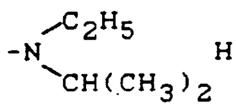 | H | 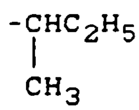 | $CH_3$ | O | (amorph) |
| 162 | 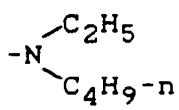 | H | $-CH(C_2H_5)_2$ | $CH_3$ | O | (amorph) |
| 163 | 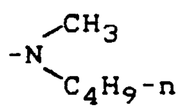 | H | $-CH_2C(CH_3)_3$ | $CH_3$ | O | (amorph) |
| 164 | 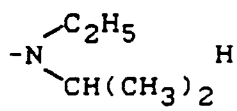 | H | $-CH_2C(CH_3)_3$ | $CH_3$ | O | (amorph) |

Tabelle 1 - Fortsetzung

| Bsp.-Nr. | 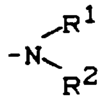 | $R^3$ | $R^4$ | $R^5$ | X | Schmelzpunkt (°C) bzw. $^1H$-NMR |
|---|---|---|---|---|---|---|
| 165 | 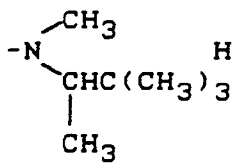 | H | 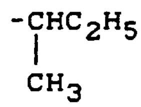 | $CH_3$ | O | (amorph) |
| 166 | 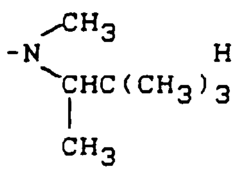 | H | 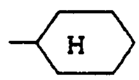 | $CH_3$ | O | (amorph) |
| 167 | 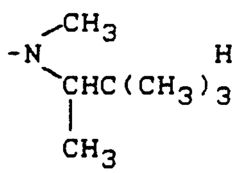 | H | $-CH_2C(CH_3)_3$ | $CH_3$ | O | (amorph) |
| 168 | 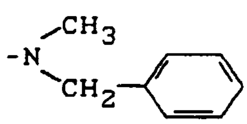 | H | $-CH_2CH(CH_3)_2$ | $CH_3$ | O | (amorph) |
| 169 | 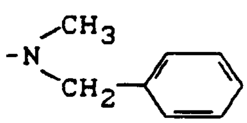 | H | 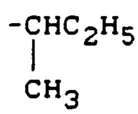 | $CH_3$ | O | 72° C |
| 170 | 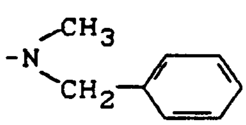 | H | 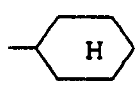 | $CH_3$ | O | 101° C |

Tabelle 1 - Fortsetzung

| Bsp.-Nr. | 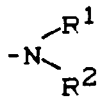 | $R^3$ | $R^4$ | $R^5$ | X | Schmelzpunkt (°C) bzw. $^1$H-NMR |
|---|---|---|---|---|---|---|
| 171 | 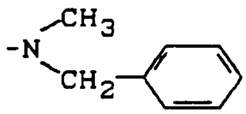 | H | $-CH_2C(CH_3)$ | $CH_3$ | O | 106° C |
| 172 | 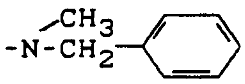 | H | $-CH(CH_3)_2$ | $CH_3$ | O | 87° C |
| 173 | 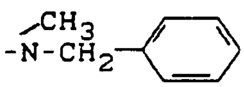 | H | 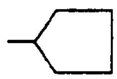 | $CH_3$ | O | 103° C |
| 174 | 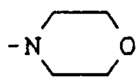 | H | 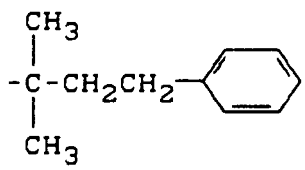 | $CH_3$ | O | 93° C |
| 175 | 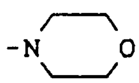 | H | 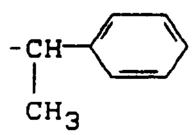 [S(-)] | $CH_3$ | O | (amorph) |
| 176 | 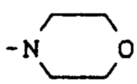 | H | 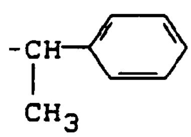 [R(+)] | $CH_3$ | O | (amorph) |

Tabelle 1 - Fortsetzung

| Bsp.-Nr. | 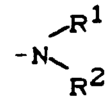 | $R^3$ | $R^4$ | $R^5$ | X | Schmelzpunkt (°C) bzw. $^1$H-NMR |
|---|---|---|---|---|---|---|
| 177 | 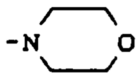 | H | 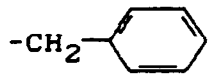 | $CH_3$ | O | 149°C |
| 178 | 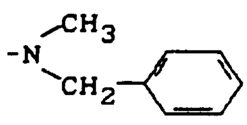 | H | 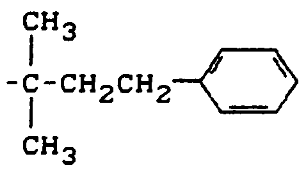 | $CH_3$ | O | (amorph) |
| 179 | 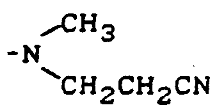 | H | $-CH_2CH(CH_3)_2$ | $CH_3$ | O | (amorph) |
| 180 | 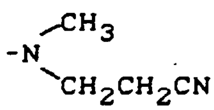 | H | 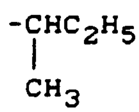 | $CH_3$ | O | (amorph) |

*) Die $^1$H-NMR-Spektren wurden in $CDCl_3$ mit Tetramethylsilan (TMS) als innerem Standard aufgenommen. Die chemische Verschiebung ist als δ-Wert in ppm angegeben.

Ausgangsstoffe der Formel (II):

Beispiel (II-1)

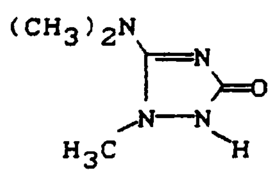

1. Stufe:

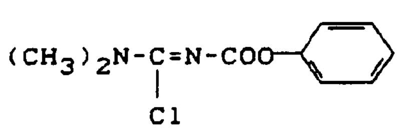

Eine Mischung aus 35 g (0,5 Mol) Dimethylcyanamid und 156 g (1,0 Mol) Chlorameisensäure-phenylester wird 16 Stunden bei 100°C gerührt. Dann werden leichter flüchtige Komponenten im Wasserstrahlvakuum (Badtemperatur: 80°C) abdestilliert und der ölige Rückstand wird im Ölpumpenvakuum destilliert.

Man erhält 61,4 g (54% der Theorie) N,N-Dimethyl-N'-phenoxycarbonyl-chlorformamidin vom Siedebereich 158°C-165°C/0,5 mbar - 0,8 mbar.

2. Stufe:

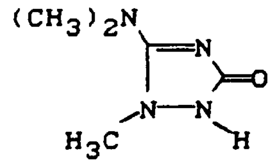

Eine Lösung von 6,9 g (0,15 Mol) Methylhydrazin in 100 ml Dioxan wird bei 20°C bis 30°C (Kühlung erforderlich!) zu einer Mischung aus 17,0 g (0,075 Mol) N,N-Dimethyl-N'-phenoxycarbonyl-chlorformamidin und 100 ml Methylenchlorid unter Rühren tropfenweise gegeben. Das Reaktionsgemisch wird 4 Stunden bei 20°C gerührt und anschließend abgesaugt. Vom Filtrat wird das Lösungsmittel im Wasserstrahlvakuum und das freigesetzte Phenol im Ölpumpenvakuum abdestilliert.

Man erhält 9,8 g (92% der Theorie) 5-Dimethylamino-1-methyl-1,2-dihydro-3H-1,2,4-triazol-3-on als öligen Rückstand.

$^1$H-NMR (DMSO-d6, δ, ppm): 3,20; 3,45.

Das Produkt kann durch Aufkochen mit Acetonitril zur Kristallisation gebracht werden; Schmelzpunkt: 222°C.

Beispiel (II-2)

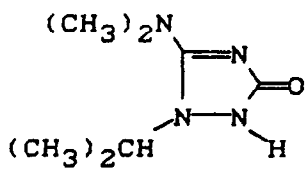

1. Stufe:

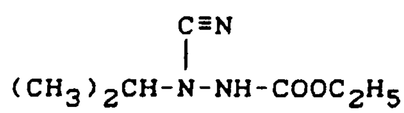

73 g (0,5 Mol) 2-Isopropylhydrazincarbonsäureethylester und 42 g (0,5 Mol) Natriumhydrogencarbonat werden in 200 ml Methylenchlorid und 400 ml Wasser eingetragen. Unter Rühren werden bei Raumtemperatur (Kühlung erforderlich) 55 g (0,52 Mol) Bromcyan eingetragen. Nach dreistündigem Nachrühren ist die $CO_2$-Entwicklung beendet. Die organische Phase wird im Scheidetrichter abgetrennt, über Natriumsulfat getrocknet und im Vakuum einrotiert.

Als Rückstand werden 73 g (85,4% d.Th.) 2-Isopropyl-2-cyanohydrazincarbonsäure-ethylester als helles Öl erhalten.

$^1$H-NMR: ($CDCl_3$, δ, ppm) 1,25-1,30; 1,30-1,35; 3,5-3,65; 4,20-4,30.

2. Stufe:

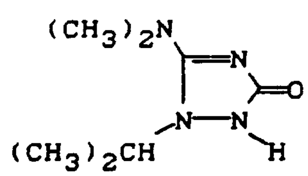

In eine Lösung von 100 ml 33%igem Dimethylamin in absolutem Ethanol werden 35 g (0,2 Mol) 2-Isopropyl-

2-cyanohydrazincarbonsäure-ethylester eingetropft. Die Lösung wird zunächst 8 Stunden bei Raumtemperatur und danach 1,5 Stunden bei Rückflußtemperatur gerührt. Nach dem Einrotieren im Vakuum bleibt ein öliger Rückstand, der beim Stehen langsam kristallisiert. Durch Umkristallisation aus Cyclohexan/Essigsäureethylester (Vol. 4:1) erhält man 30 g (88% der Theorie) 5-Dimethylamino-1-isopropyl-1,2-dihydro-3H-1,2,4-triazol-3-on vom Schmelzpunkt 111°C.

Analog können beispielsweise die in der nachstehenden Tabelle 2 aufgeführten Verbindungen der Formel (II) hergestellt werden:

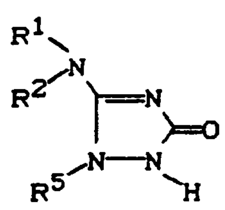

(II)

Tabelle 2: Beispiele für die Ausgangsstoffe der Formel (II)

| Bsp.-Nr. | $R^1$ | $R^2$ | $R^5$ | Schmelzpunkt (°C) |
|---|---|---|---|---|
| II-3 | $C_2H_5$ | $C_2H_5$ | $CH_3$ | 89 |
| II-4 | $-CH_2CH_2-O-CH_2CH_2-$ | | $CH_3$ | 183 |
| II-5 | $-(CH_2)_4-$ | | $CH_3$ | (amorph) |
| II-6 | $-(CH_2)_5-$ | | $CH_3$ | (amorph) |
| II-7 | $CH(CH_3)_2$ | $CH(CH_3)_2$ | $CH_3$ | 119 |
| II-8 | $CH_3$ | $C_2H_5$ | $CH_3$ | (amorph) |
| II-9 | $CH_3$ | $CH_3$ | $C_2H_5$ | |

Ausgangsstoffe der Formel (IV):

Beispiel (IV-1)

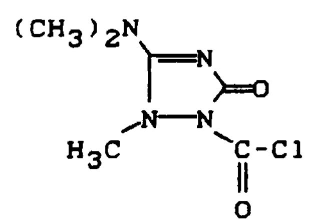

Eine Mischung aus 14,2 g (0,1 Mol) 5-Dimethylamino-1-methyl-1,2-dihydro-3H-1,2,4-triazol-3-on und 200 ml Acetonitril wird unter Einleiten von Phosgen auf Rückflußtemperatur erhitzt und bis zum Ende der Chlorwasserstoffentwicklung phosgeniert. Anschließend wird Stickstoff durch die Mischung geblasen und dann das Lösungsmittel im Wasserstrahlvakuum sorgfältig abdestilliert.

Man erhält 18,5 g (91% der Theorie) 2-Chlorcarbonyl-5-dimethylamino-1-methyl-1,2-dihydro-3H-1,2,4-triazol-3-on als öligen Rückstand, der ohne weitere Reinigung bzw. Charakterisierung für die nächste Stufe gemäß Verfahren (b) eingesetzt wird.

Anwendungsbeispiele

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine ausgezeichnete herbizide Wirksamkeit zeigen in diesem Test z.B. die Verbindungen gemäß den Herstellungsbeispielen: 1, 5, 7, 9, 10, 11, 12, 13, 14, 16, 20, 21, 22, 23, 24, 25, 32, 34, 49 und 54.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Eine ausgezeichnete herbizide Wirksamkeit bei guter bis sehr guter Nutzpflanzenselektivität zeigen in diesem Test z.B. die Verbindungen gemäß den Herstellungsbeispielen 1, 2, 4, 5, 9, 10, 11, 12, 13, 14, 20, 21, 22, 23, 24, 25, 26, 28, 29, 32, 34, 35 und 36.

## Patentansprüche

1. Substituierte Triazolinone der Formel (I),

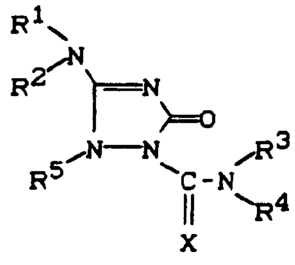

(I)

in welcher

| | |
|---|---|
| $R^1$ und $R^2$ | unabhängig voneinander jeweils für Alkyl, Alkenyl, Alkinyl, Halogenalkyl, Halogenalkenyl, Halogenalkinyl, Cyanalkyl, Alkoxyalkyl, Alkoxy, Cycloalkyl, Cycloalkylalkyl, für jeweils gegebenenfalls substituiertes Aryl, Aralkyl oder Heteroaryl stehen oder gemeinsam mit dem Stickstoffatom, an welches sie gebunden sind, für einen gegebenenfalls substituierten Heterocyclus stehen, |
| $R^3$ und $R^4$ | unabhängig voneinander jeweils für Wasserstoff, Alkyl, Alkenyl, Alkinyl, Halogenalkyl, Halogenalkenyl, Halogenalkinyl, Cyanalkyl, Hydroxyalkyl, Alkoxyalkyl, Alkylaminoalkyl, Dialkylaminoalkyl, Alkoxycarbonylalkyl, Alkoxycarbonylalkenyl, für jeweils gegebenenfalls substituiertes Cycloalkyl, Cycloalkylalkyl, Cycloalkenyl oder Cycloalkenylalkyl, für gegebenenfalls substituiertes Heterocyclylalkyl, für jeweils gegebenenfalls substituiertes Aralkyl, Aroyl oder Aryl, für Alkoxy, Alkenyloxy, Alkinyloxy, Aralkyloxy oder Aryloxy stehen oder gemeinsam mit dem Stickstoffatom, an welches sie gebunden sind, für einen gegebenenfalls substituierten Heterocyclus stehen, |
| $R^5$ | für Alkyl oder Cycloalkyl steht und |
| X | für Sauerstoff oder Schwefel steht. |

2. Substituierte Triazolinone der Formel (I) gemäß Anspruch 1, in welcher

| | |
|---|---|
| $R^1$ und $R^2$ | unabhängig voneinander für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl, Alkoxyalkyl oder Alkoxy mit jeweils 1 bis 6 Kohlenstoffatomen in den einzelnen Alkylteilen, für Cycloalkyl mit 3 bis 7 Kohlenstoffatomen, für Cycloalkylalkyl mit 3 bis 7 Kohlenstoffatomen im Cycloalkylteil und 1 bis 6 Kohlenstoffatomen im Alkylteil oder für jeweils gegebenenfalls einfach oder mehrfach, gleich oder verschieden substituiertes Aralkyl mit 6 bis 10 Kohlenstoffatomen im Arylteil und 1 bis 6 Kohlenstoffatomen im Alkylteil, Aryl mit 6 bis 10 Kohlenstoffatomen oder Heteroaryl mit 2 bis 9 Kohlenstoffatomen und 1 bis 3 Heteroatomen, insbesondere Stickstoff, Sauerstoff und/oder Schwefel stehen, wobei als Substituenten jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen oder |
| $R^1$ und $R^2$ | gemeinsam mit dem Stickstoffatom, an welches sie gebunden sind, für einen gegebenenfalls einfach oder mehrfach gleich oder verschieden substituierten fünf- bis zehngliedrigen Heterocyclus stehen, der gegebenenfalls 1 bis 2 weitere Heteroatome, insbesondere Stickstoff, Sauerstoff und/oder Schwefel enthalten kann, wobei als Substituenten infrage kommen: Halogen sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen sowie 1 bis 2 Oxo- oder Thionogruppen, |
| $R^3$ und $R^4$ | unabhängig voneinander für Wasserstoff, für jeweils geradkettiges oder verzweigtes Alkyl mit 1 bis 18 Kohlenstoffatomen, Alkenyl mit 2 bis 8 Kohlenstofftomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, Alkoxyalkyl, Alkoxycarbonylalkyl oder Alkoxycarbonylalkenyl mit jeweils bis zu 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen oder für jeweils gegebenenfalls einfach oder mehrfach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im Alkylteil stehen, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenene Halogenatomen oder jeweils zweifach verknüpftes Alkandiyl bzw. Alkendiyl mit jeweils bis zu 4 Kohlenstoffatomen; außerdem $R^3$ und $R^4$ unabhängig von- |

einander für im Heterocyclylteil gegebenenfalls einfach oder mehrfach, gleich oder verschieden substituiertes Heterocyclylalkyl mit 1 bis 6 Kohlenstoffatomen im geradkettigen oder verzweigten Alkylteil und 1 bis 9 Kohlenstoffatomen sowie 1 bis 3 Heteroatomen - insbesondere Stickstoff, Sauerstoff und/oder Schwefel - im Heterocyclylteil stehen, wobei als Substituenten infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen; außerdem $R^3$ und $R^4$ unabhängig voneinander für jeweils geradkettiges oder verzweigtes Alkoxy mit 1 bis 8 Kohlenstoffatomen, Alkenyloxy mit 2 bis 8 Kohlenstoffatomen oder Alkinyloxy mit 2 bis 8 Kohlenstoffatomen oder für jeweils gegebenenfalls einfach oder mehrfach, gleich oder verschieden substituiertes Aralkyl, Aralkyloxy, Aryloxy, Aroyl oder Aryl mit jeweils 6 bis 10 Kohlenstoffatomen im Arylteil und gegebenenfalls 1 bis 6 Kohlenstoffatomen im Alkylteil stehen, wobei als Arylsubstituenten jeweils infrage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen und gegebenenfalls 1 bis 9 gleichen oder verschiedenen Halogenatomen, Cycloalkyl mit 3 bis 6 Kohlenstoffatomen oder Phenoxy und wobei als Alkylsubstituenten gegebenenfalls infrage kommen: Halogen oder Cyano, oder

$R^3$ und $R^4$ gemeinsam mit dem Stickstoffatom, an welches sie gebunden sind, für einen gegebenenfalls einfach oder mehrfach, gleich oder verschieden substituierten fünf- bis zehngliedrigen Heterocyclus stehen, der gegebenenfalls 1 bis 2 weitere Heteroatome, insbesondere Stickstoff, Sauerstoff und/oder Schwefel, enthalten kann, wobei als Substituenten infrage kommen: Halogen sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen sowie 1 bis 2 Oxo-oder Thionogruppen,

$R^5$ für geradkettiges oder verzweigtes Alkyl mit 1 bis 8 Kohlenstoffatomen oder Cycloalkyl mit 3 bis 8 Kohlenstoffatomen steht und

X für Sauerstoff oder Schwefel steht.

3. Substituierte Triazolinone der Formel (I) gemäß Anspruch 1, in welcher

$R^1$ und $R^2$ unabhängig voneinander jeweils für Methyl, Ethyl, n- oder i-Propyl, n-, i-, s- oder t-Butyl, n- oder i-Pentyl, Allyl, Propargyl, für jeweils geradkettiges oder verzweigtes Halogenalkyl mit 1 bis 4 Kohlenstoffatomen, Halogenalkenyl mit 3 bis 6 Kohlenstoffatomen oder Halogenalkinyl mit 3 bis 6 Kohlenstoffatomen und jeweils 1 bis 9 gleichen oder verschiedenen Halogenatomen, für Cyanethyl, Methoxymethyl, Methoxyethyl, Dimethoxyethyl, Methoxy, Ethoxy, für Cyclopropyl, Cyclopropylmethyl, Cyclopentyl, Cyclohexyl, Cyclohexylmethyl, Cyclohexylethyl, Cyclopentylmethyl oder für jeweils gegebenenfalls ein- bis dreifach, gleich oder verschieden substituiertes Benzyl, Phenylethyl oder Phenyl stehen, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio oder

$R^1$ und $R^2$ gemeinsam mit dem Stickstoffatom, an welches sie gebunden sind, für einen gegebenenfalls ein- bis dreifach, gleich oder verschieden substituierten Heterocyclus der Formel

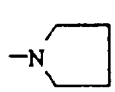 ; 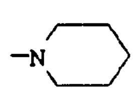 ; 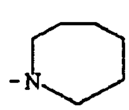 ; 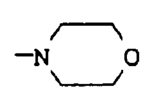 ;

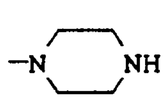 ; 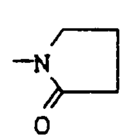 ; 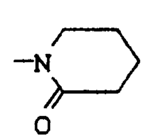 ; 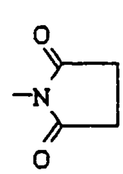 ;

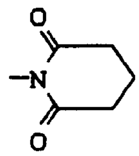 ; 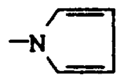 oder 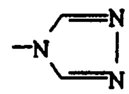

stehen,
wobei als Substituenten infrage kommen: Methyl, Ethyl, n- oder i-Propyl, Chlor oder Trifluormethyl,

$R^3$ für Wasserstoff oder Methyl steht,

$R^4$ für Methyl, Ethyl, n- oder i-Propyl, n-, i-, s- oder t-Butyl, jeweils geradkettiges oder verzweigtes Pentyl, Hexyl, Heptyl, Octyl, Nonyl, Decyl oder Dodecyl, für Allyl, jeweils geradkettiges der verzweigtes Butenyl, Pentenyl, Hexenyl, Propargyl, Butinyl, Pentinyl oder Hexinyl, für geradkettiges oder verzweigtes Halogenalkyl mit 1 bis 6 Kohlenstoffatomen und 1 bis 9 gleichen oder verschiedenen Halogenatomen, für jeweils geradkettiges oder verzweigtes Halogenalkenyl oder Halogenalkinyl mit jeweils 3 bis 5 Kohlenstoffatomen und 1 bis 3 Halogenatomen, für jeweils geradkettiges oder verzweigtes Cyanalkyl mit 1 bis 6 Kohlenstoffatomen im Alkylteil, Hydroxyalkyl mit 1 bis 6 Kohlenstoffatomen und 1 bis 3 Hydroxygruppen, Alkoxyalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl- und Alkenylteilen oder für jeweils gegebenenfalls ein- bis dreifach, gleich oder verschieden substituiertes Cyclopropyl, Cyclopropylmethyl, Cyclopropylethyl, Cyclopentyl, Cyclohexyl, Cycloheptyl, Cyclooctyl, Cyclohexylmethyl, Cyclohexylethyl, Cyclohexenyl oder Cyclohexenylmethyl steht, wobei als Substituenten jeweils infrage kommen: Fluor, Chlor, Brom, Methyl, Ethyl, n- oder i-Propyl, n-, i-, s- oder t-Butyl, Cyano, Methandiyl, Ethandiyl, Butandiyl oder Butadiendiyl;
außerdem für im Heterocyclylteil gegebenenfalls ein- bis dreifach, gleich oder verschieden substituiertes Heterocyclylmethyl, Heterocyclylpropyl oder Heterocyclylethyl steht, wobei als Heterocyclen jeweils infrage kommen:

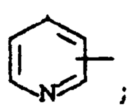 ; 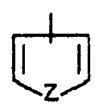 ; 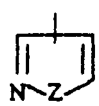 ; 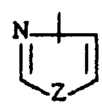 ;

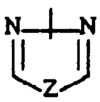 ; 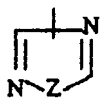 ; 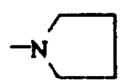 ; 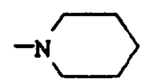 ;

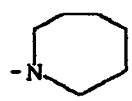 ; 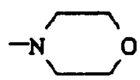 oder 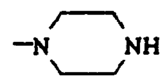

wobei Z jeweils für Sauerstoff oder Schwefel steht und wobei als Substituenten infrage kommen:
Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio; $R^4$ außerdem für jeweils geradkettiges oder verzweigtes Alkoxy mit 1 bis 6 Kohlenstoffatomen, Alkenyloxy mit 3 bis 6 Kohlenstoffatomen oder Alkinyloxy mit 3 bis 6 Kohlenstoffatomen oder für jeweils gegebenenfalls ein- bis dreifach, gleich oder verschieden substituiertes, gegebenenfalls geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl, Phenylcyanpropyl, Benzyloxy, Phenylethyloxy, Phenoxy, Benzoyl, Phenyl oder Naphthyl steht, wobei als Phenylsubstituenten jeweils infrage kommen: Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Trifuormethylsulfinyl, Trifluormethylsulfonyl, Methylsulfinyl, Methylsulfonyl, Acetyl, Propionyl, Methoxycarbonyl, Ethoxycarbonyl, Cyclohexyl oder Phenoxy; oder

$R^3$ und $R^4$ gemeinsam mit dem Stickstoffatom, an welches sie gebunden sind, für einen gegebenenfalls ein- bis dreifach, gleich oder verschieden substituierten Heterocyclus der Formel

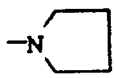 ; 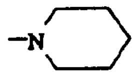 ; 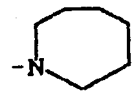 ; 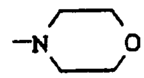 ;

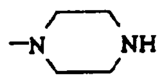 ; 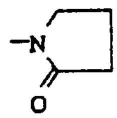 ; 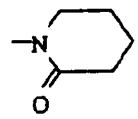 ; 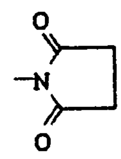 ;

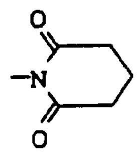 ; 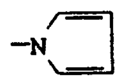 oder 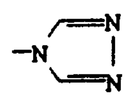

stehen,

wobei als Substituenten infrage kommen: Methyl, Ethyl, n- oder i-Propyl, Chlor oder Trifluormethyl,

$R^5$ für Methyl, Ethyl, Propyl, Isopropyl, n-, i-, s-oder t-Butyl, Cyclopropyl, Cyclobutyl, Cyclopentyl oder Cyclohexyl steht und

X für Sauerstoff oder Schwefel steht.

4. Verfahren zur Herstellung von substituierten Triazolinonen der Formel (I)

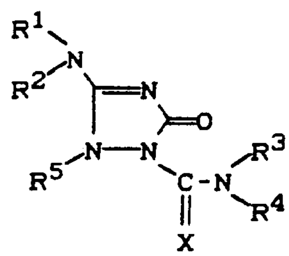

(I)

in welcher

$R^1$ und $R^2$ unabhängig voneinander jeweils für Alkyl, Alkenyl, Alkinyl, Halogenalkyl, Halogenalkenyl, Halogenalkinyl, Cyanalkyl, Alkoxyalkyl, Alkoxy, Cycloalkyl, Cycloalkylalkyl, für jeweils gegebenenfalls substituiertes Aryl, Aralkyl oder Heteroaryl stehen oder gemeinsam mit dem Stickstoffatom, an welches sie gebunden sind, für einen gegebenenfalls substituierten Heterocyclus stehen,

$R^3$ und $R^4$ unabhängig voneinander jeweils für Wasserstoff, Alkyl, Alkenyl, Alkinyl, Halogenalkyl, Halogenalkenyl, Halogenalkinyl, Cyanalkyl, Hydroxyalkyl, Alkoxyalkyl, Alkylaminoalkyl, Dialkylaminoalkyl, Alkoxycarbonylalkyl, Alkoxycarbonylalkenyl, für jeweils gegebenenfalls substituiertes Cycloalkyl, Cycloalkylalkyl, Cycloalkenyl oder Cycloalkenylalkyl, für gegebenenfalls substituiertes Heterocyclylalkyl, für jeweils gegebenenfalls substituiertes Aralkyl, Aroyl oder Aryl, für Alkoxy, Alkenyloxy, Alkinyloxy, Aralkyloxy oder Aryloxy stehen oder gemeinsam mit dem Stickstoffatom, an welches sie gebunden sind, für einen gegebenenfalls substituierten Heterocyclus stehen,

$R^5$ für Alkyl oder Cycloalkyl steht und
X für Sauerstoff oder Schwefel steht,
dadurch gekennzeichnet, daß man
a) Triazolinone der Formel (II)

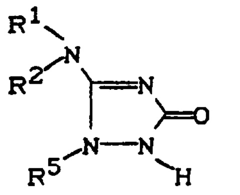

(II)

in welcher
$R^1$, $R^2$ und $R^5$ die oben angegebene Bedeutung haben,
und/oder Tautomere dieser Verbindungen,
mit Iso(thio)cyanaten der allgemeinen Formel (III)

$$R^4\text{-N=C=X} \quad \text{(III)}$$

in welcher
$R^4$ und X die oben angegebene Bedeutung haben,
gegebenenfalls in Gegenwart eines Reaktionshilfsmittels und gegebenenfalls in Gegenwart eines Verdünnungsmittels umsetzt, oder daß man
b) Chlorcarbonyl-triazolinone der allgemeinen Formel (IV)

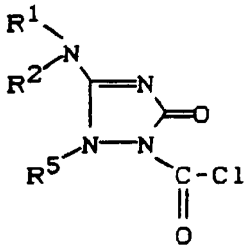

(IV)

in welcher
$R^1$, $R^2$ und $R^5$ die oben angegebene Bedeutung haben,
mit Aminen der allgemeinen Formel (V)

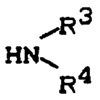

(V)

in welcher
$R^3$ und $R^4$ die oben angegebene Bedeutung haben,
gegebenenfalls in Gegenwart eines Säurebindemittels und gegebenenfalls in Gegenwart eines Verdünnungsmittels umsetzt.

5. Herbizide Mittel, gekennzeichnet durch einen Gehalt an mindestens einem substiutierten Triazolinon der Formel (I) gemäß Anspruch 1 oder 4.

6. Verfahren zur Bekämpfung von unerwünschten Pflanzen, dadurch gekennzeichnet, daß man substituierte Triazolinone der Formel (I) gemäß Anspruch 1 oder 4 auf die Pflanzen und/oder ihren Lebensraum einwirken läßt.

7. Verwendung von substituierten Triazolinonen der Formel (I) gemäß Anspruch 1 oder 4 zur Bekämpfung von unerwünschten Pflanzen.

8. Verfahren zur Herstellung von herbiziden Mitteln, dadurch gekennzeichnet, daß man substituierte Triazolinone der Formel (I) gemäß Anspruch 1 oder 4 mit Streckmitteln und/oder oberflächenaktiven Substanzen vermischt.

9. Chlorcarbonyl-triazolinone der Formel (IV)

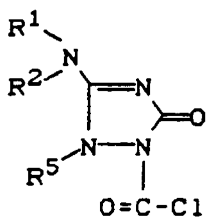

IV

in welcher

| | |
|---|---|
| $R^1$ und $R^2$ | unabhängig voneinander jeweils für Alkyl, Alkenyl, Alkinyl, Halogenalkyl, Halogenalkenyl, Halogenalkinyl, Cyanalkyl, Alkoxyalkyl, Alkoxy, Cycloalkyl, Cycloalkylalkyl, für jeweils gegebenenfalls substituiertes Aryl, Aralkyl oder Heteroaryl stehen oder gemeinsam mit dem Stickstoffatom, an welches sie gebunden sind, für einen gegebenenfalls substituierten Heterocyclus stehen und |
| $R^5$ | für Alkyl oder Cycloalkyl steht. |

## Claims

1. Substituted triazolinones of the formula (I)

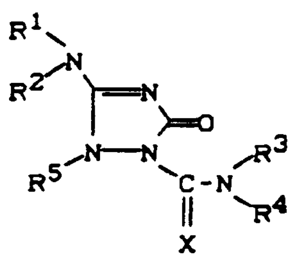

(I)

in which

| | |
|---|---|
| $R^1$ and $R^2$, | independently of one another, each represent alkyl, alkenyl, alkinyl, halogenoalkyl, halogenoalkenyl, halgenoalkinyl, cyanoalkyl, alkoxyalkyl, alkoxy, cycloalkyl, cycloalkylalkyl, represent aryl, aralkyl or heteroaryl, each of which is optionally substituted, or, together with the nitrogen atom to which they are bonded, represent an optionally substituted heterocycle, |
| $R^3$ and $R^4$, | independently of one another, each represent hydrogen, alkyl, alkenyl, alkinyl, halogenoalkyl, halogenoalkenyl, halogenoalkinyl, cyanoalkyl, hydroxyalkyl, alkoxyalkyl, alkylaminoalkyl, dialkylaminoalkyl, alkoxycarbonylalkyl, alkoxycarbonylalkenyl, represent cycloalkyl, cycloalkylalkyl, cycloalkenyl or cycloalkenylalkyl, each of which is optionally substituted, represent optionally substituted heterocyclylalkyl, represent aralkyl, aroyl or aryl, each of which is optionally substituted, represent alkoxy, alkenyloxy, alkinyloxy, aralkyloxy or aryloxy, or, together with the nitrogen to which they are bonded, represent an optionally substituted heterocycle, |
| $R^5$ | represents alkyl or cycloalkyl and |
| X | represents oxygen or sulphur. |

2. Substituted triazolinones of the formula (I) according to Claim 1,
in which

| | |
|---|---|
| $R^1$ and $R^2$, | independently of one another, represent alkyl having 1 to 8 carbon atoms, alkenyl hav- |

ing 2 to 8 carbon atoms, alkinyl having 2 to 8 carbon atoms, halogenoalkyl having 1 to 8 carbon atoms and 1 to 17 identical or different halogen atoms, halogenoalkenyl or halogenoalkinyl each having 2 to 8 carbon atoms and 1 to 15 or 13 identical or different halogen atoms respectively, cyanoalkyl, alkoxyalkyl or alkoxy each having 1 to 6 carbon atoms in the individual alkyl moieties, each of the preceding species being straight-chain or branched, represent cycloalkyl having 3 to 7 carbon atoms, represent cycloalkylalkyl having 3 to 7 carbon atoms in the cycloalkyl moiety and 1 to 6 carbon atoms in the alkyl moiety or represent aralkyl having 6 to 10 carbon atoms in the aryl moiety and 1 to 6 carbon atoms in the alkyl moiety, aryl having 6 to 10 carbon atoms or heteroaryl having 2 to 9 carbon atoms and 1 to 3 hetero atoms, in particular nitrogen, oxygen and/or sulphur, each of these species being optionally mono- or poly-substituted by identical or different substituents, suitable substituents in each case being: halogen, cyano, nitro and also alkyl, alkoxy, alkylthio, halogenoalkyl, halogenoalkoxy or halogenoalkylthio, each of which may be straight-chain or branched, each having 1 to 4 carbon atoms and, if appropriate, 1 to 9 identical or different halogen atoms or

$R^1$ and $R^2$, together with the nitrogen atom to which they are bonded, represent a 5- to 10-membered heterocycle which is optionally mono- or poly-substituted by identical or different substituents and may optionally contain 1 to 2 other hetero atoms, in particular nitrogen, oxygen and/or sulphur, suitable substituents being: halogen and also alkyl or halogenoalkyl, each of which may be straight-chain or branched, each having 1 to 4 carbon atoms and, if appropriate, 1 to 9 identical or different halogen atoms and also 1 to 2 oxo or thiono groups,

$R^3$ and $R^4$, independently of one another, represent hydrogen, represent alkyl having 1 to 18 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl or halogenoalkinyl, each having 2 to 8 carbon atoms and 1 to 15 or 13 identical or different halogen atoms respectively, cyanoalkyl having 1 to 8 carbon atoms, hydroxyalkyl having 1 to 8 carbon atoms and 1 to 6 hydroxyl groups, alkoxyalkyl, alkoxycarbonylalkyl or alkoxycarbonylalkenyl, each having up to 6 carbon atoms in the individual alkyl or alkenyl moieties, alkylaminoalkyl or dialkylaminoalkyl, each having 1 to 6 carbon atoms in the individual alkyl moieties, each of these species being straight-chain or branched, or represent cycloalkyl, cycloalkylalkyl, cycloalkenyl or cycloalkenylalkyl, each having 3 to 8 carbon atoms in the cycloalkyl or cycloalkenyl moiety and, if appropriate, 1 to 6 carbon atoms in the alkyl moiety, each of which is optionally mono- or poly-substituted by identical or different substituents, suitable substituents in each case being: halogen, cyano and also alkyl or halogenoalkyl, each of which may be straight-chain or branched, each having 1 to 4 carbon atoms and, if appropriate, 1 to 9 identical or different halogen atoms or alkanediyl or alkenediyl, each of which is doubly linked, each having up to 4 carbon atoms; furthermore $R^3$ and $R^4$, independently of one another, represent heterocyclylalkyl having 1 to 6 carbon atoms in the straight-chain or branched alkyl moiety and 1 to 9 carbon atoms and 1 to 3 hetero atoms in the heterocyclyl moiety, in particular nitrogen, oxygen and/or sulphur, the heterocyclyl moiety being optionally mono- or poly-substituted by identical or different substituents, suitable substituents being: halogen, cyano, nitro and also alkyl, alkoxy, alkylthio, halogenoalkyl, halogenoalkoxy, halogenoalkylthio or alkoxycarbonyl, each of which is straight-chain or branched, each having 1 to 5 carbon atoms and, if appropriate, 1 to 9 identical or different halogen atoms; furthermore, $R^3$ and $R^4$, independently of one another, represent alkoxy having 1 to 8 carbon atoms, alkenyloxy having 2 to 8 carbon atoms or alkinyloxy having 2 to 8 carbon atoms, each being straight-chain or branched, or represent aralkyl, aralkyloxy, aryloxy, aroyl or aryl, each having 6 to 10 carbon atoms in the aryl moiety and, if appropriate, 1 to 6 carbon atoms in the alkyl moiety, each of which is optionally mono- or poly-substituted by identical or different substituents, suitable aryl substituents in each case being: halogen, cyano, nitro, hydroxyl and also alkyl, alkoxy, alkylthio, halogenoalkyl, halogenoalkoxy, halogenoalkylthio, alkylsulphinyl, alkylsulphonyl, halogenoalkylsulphinyl, halogenoalkylsulphonyl, alkanoyl or alkoxycarbonyl, each of which may be straight-chain or branched, each having 1 to 6 carbon atoms and, if appropriate, 1 to 9 identical or different halogen

atoms, cycloalkyl having 3 to 6 carbon atoms or phenoxy and suitable alkyl substituents, if desired, being: halogen or cyano, or

$R^3$ and $R^4$, together with the nitrogen atom to which they are bonded, represent a 5- to 10-membered heterocycle which is optionally mono- or poly-substituted by identical or different substituents and optionally can contain 1 to 2 other hetero atoms, in particular nitrogen, oxygen and/or sulphur, suitable substituents being: halogen and also alkyl or halogenoalkyl, each of which may be straight-chain or branched, each having 1 to 4 carbon atoms and, if appropriate, 1 to 9 identical or different halogen atoms, and also 1 to 2 oxo or thiono groups,

$R^5$ represents straight-chain or branched alkyl having 1 to 8 carbon atoms or cycloalkyl having 3 to 8 carbon atoms and

X represents oxygen or sulphur.

3. Substituted triazolinones of the formula (I) according to Claim 1, in which

$R^1$ and $R^2$, independently of one another, each represent methyl, ethyl, n- or i-propyl, n-, i-, s- or t-butyl, n- or i-pentyl, allyl, propargyl, represent halogenoalkyl having 1 to 4 carbon atoms, halogenoalkenyl having 3 to 6 carbon atoms or halogenoalkinyl having 3 to 6 carbon atoms and in each case having 1 to 9 identical or different halogen atoms, each of these species being straight-chain or branched, represent cyanoethyl, methoxymethyl, methoxyethyl, dimethoxyethyl, methoxy, ethoxy, represent cyclopropyl, cyclopropylmethyl, cyclopentyl, cyclohexyl, cyclohexylmethyl, cyclohexylethyl, cyclopentylmethyl or represent benzyl, phenylethyl or phenyl, each of which is optionally mono- to tri-substituted by identical or different substituents, suitable substituents being: fluorine, chlorine, bromine, cyano, nitro, methyl, ethyl, n- or i-propyl, n-, i-, s- or t-butyl, methoxy, ethoxy, methylthio, trifluoromethyl, trifluoromethoxy or trifluoromethylthio or

$R^1$ and $R^2$, together with the nitrogen atom to which they are bonded, represent a heterocycle of the formula

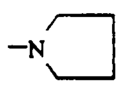 ; 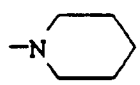 ; 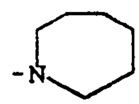 ; 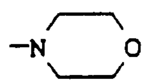 ;

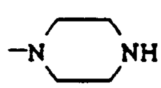 ; 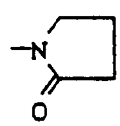 ; 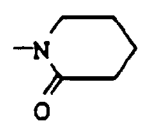 ; 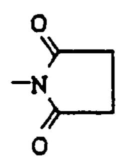 ;

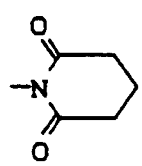 ; 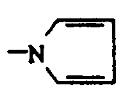 or 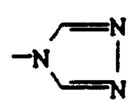

which is optionally mono- to tri-substituted by identical or different substituents, suitable substituents being: methyl, ethyl, n- or i-propyl, chlorine or trifluoromethyl,

$R^3$ represents hydrogen or methyl,

$R^4$ represents methyl, ethyl, n- or i-propyl, n-, i-, s- or t-butyl, represents pentyl, hexyl, heptyl, octyl, nonyl, decyl or dodecyl, each of which is straight-chain or branched, represents allyl, represents butenyl, pentenyl, hexenyl, propargyl, butinyl, pentinyl or hexinyl, each of which is straight-chain or branched, represents straight-chain or branched halogenoalkyl having 1 to 6 carbon atoms and 1 to 9 identical or different halogen atoms, represents halogenoalkenyl or halogenoalkinyl, each of which is straight-chain

or branched, each having 3 to 5 carbon atoms and 1 to 3 halogen atoms, represents cyanoalkyl having 1 to 6 carbon atoms in the alkyl moiety, hydroxyalkyl having 1 to 6 carbon atoms and 1 to 3 hydroxyl groups, alkoxyalkyl, alkoxycarbonylalkyl or alkoxycarbonylalkenyl, alkylaminoalkyl or dialkylaminoalkyl, each of which is straight-chain or branched, each having up to 4 carbon atoms in the individual alkyl and alkenyl moieties, or represents cyclopropyl, cyclopropylmethyl, cyclopropylethyl, cyclopentyl, cyclohexyl, cycloheptyl, cyclooctyl, cyclohexylmethyl, cyclohexylethyl, cyclohexenyl or cyclohexenylmethyl, each of which is optionally mono-to tri-substituted by identical or different substituents, suitable substituents in each case being: fluorine, chlorine, bromine, methyl, ethyl, n- or i-propyl, n-, i-, s- or t-butyl, cyano, methanediyl, ethanediyl, butanediyl or butadienediyl;

furthermore represents heterocyclylmethyl, heterocyclylpropyl or heterocyclylethyl, each of which is optionally mono- to tri-substituted by identical or different substituents in the heterocyclyl moiety, suitable heterocycles in each case being:

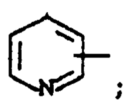 ; 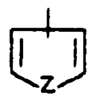 ; 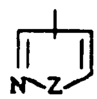 ; 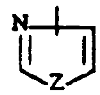 ;

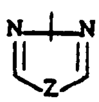 ; 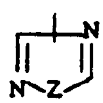 ; 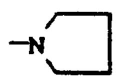 ; 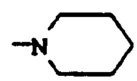 ;

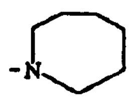 ; 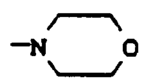 or 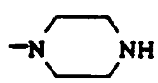

Z in each case representing oxygen or sulphur and suitable substituents being: fluorine, chlorine, bromine, cyano, nitro, methyl, ethyl, n- or i-propyl, n-, i-, s- or t-butyl, methoxy, ethoxy, methylthio, trifluoromethyl, trifluoromethoxy or trifluoromethylthio;

$R^4$ furthermore represents alkoxy having 1 to 6 carbon atoms, alkenyloxy having 3 to 6 carbon atoms or alkinyloxy having 3 to 6 carbon atoms, each of which is straight-chain or branched, or represents benzyl, phenylethyl, phenylpropyl, phenylbutyl, phenylpentyl, phenylhexyl, phenylheptyl, phenylcyanomethyl, phenylcyanoethyl, phenylcyanopropyl, benzyloxy, phenylethyloxy, phenoxy, benzoyl, phenyl or naphthyl, each of which is optionally straight-chain or branched and optionally mono- to tri-substituted by identical or different substituents, suitable phenyl substituents in each case being: fluorine, chlorine, bromine, hydroxyl, cyano, nitro, methyl, ethyl, n- or i-propyl, n-, i-, s- or t-butyl, methoxy, ethoxy, methylthio, trifluoromethyl, trifluoromethoxy, trifluoromethylthio, trifluoromethylsulphinyl, trifluoromethylsulphonyl, methylsulphinyl, methylsulphonyl, acetyl, propionyl, methoxycarbonyl, ethoxycarbonyl, cyclohexyl or phenoxy; or

$R^3$ and $R^4$, together with the nitrogen atom to which they are bonded, represent a heterocycle of the formula

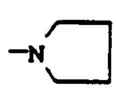 ; 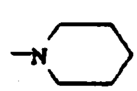 ; 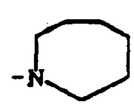 ; 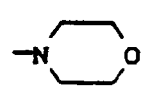 ;

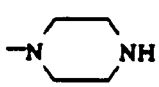 ; 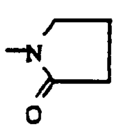 ; 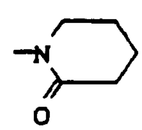 ; 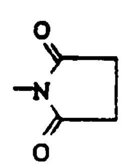 ;

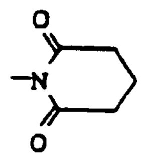 ; 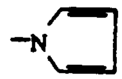 or 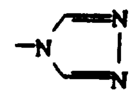

which is optionally mono- to tri-substituted by identical or different substituents, suitable substituents being: methyl, ethyl, n- or i-propyl, chlorine or trifluoromethyl,

$R^5$ represents methyl, ethyl, propyl, isopropyl, n-, i-, s- or t-butyl, cyclopropyl, cyclobutyl, cyclopentyl or cyclohexyl and

X represents oxygen or sulphur.

4. Process for the preparation of substituted triazolinones of the formula (I)

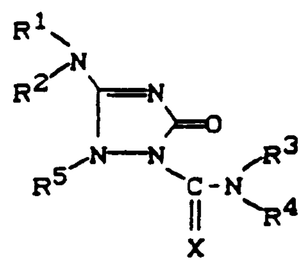 (I)

in which

$R^1$ and $R^2$, independently of one another, each represent alkyl, alkenyl, alkinyl, halogenoalkyl, halogenoalkenyl, halogenoalkinyl, cyanoalkyl, alkoxyalkyl, alkoxy, cycloalkyl, cycloalkylalkyl, represent aryl, aralkyl or heteroaryl, each of which is optionally substituted, or, together with the nitrogen atom to which they are bonded, represent an optionally substituted heterocycle,

$R^3$ and $R^4$, independently of one another, each represent hydrogen, alkyl, alkenyl, alkinyl, halogenoalkyl, halogenoalkenyl, halogenoalkinyl, cyanoalkyl, hydroxyalkyl, alkoxyalkyl, alkylaminoalkyl, dialkylaminoalkyl, alkoxycarbonylalkyl, alkoxycarbonylalkenyl, represent cycloalkyl, cycloalkylalkyl, cycloalkenyl or cycloalkenylalkyl, each of which is optionally substituted, represent optionally substituted heterocyclylalkyl, represent aralkyl, aroyl or aryl, each of which is optionally substituted, represent alkoxy, alkenyloxy, alkinyloxy, aralkyloxy or aryloxy, or, together with the nitrogen to which they are bonded, represent an optionally substituted heterocycle,

$R^5$ represents alkyl or cycloalkyl and

X represents oxygen or sulphur,

characterized in that

a) triazolinones of the formula (II)

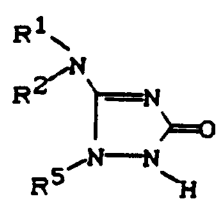 (II)

in which

$R^1$, $R^2$ and $R^5$ have the meaning given above,

and/or tautomers of these compounds,

are reacted, optionally in the presence of a reaction auxiliary and optionally in the presence of a diluent, with iso(thio)cyanates of the general formula (III)

$$R^4\text{-N=C-X} \qquad \text{(III)}$$

in which
$R^4$ and X have the meaning given above, or
b) chlorocarbonyl-triazolinones of the general formula (IV)

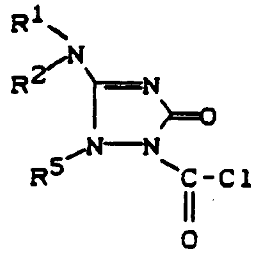

(IV)

in which $R^1$, $R^2$ and $R^5$ have the meaning given above,
are reacted, optionally in the presence of an acid-binder and optionally in the presence of a diluent, with amines of the general formula (V)

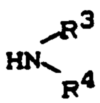

(V)

in which
$R^3$ and $R^4$ have the meaning given above.

5. Herbicidal agents, characterized in that they contain at least one substituted triazolinone of the formula (I) according to Claim 1 or 4.

6. Process for combating unwanted plants, characterized in that substituted triazolinones of the formula (I) according to Claim 1 or 4 are allowed to act on the plants and/or their habitat.

7. The use of substituted triazolinones of the formula (I) according to Claim 1 or 4 for combating unwanted plants.

8. Process for the preparation of herbicidal agents, characterized in that substituted triazolinones of the formula (I) according to Claim 1 or 4 are mixed with extenders and/or surface-active substances.

9. Chlorocarbonyl-triazolinones of the formula (IV)

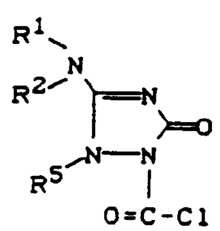

IV

in which

| | |
|---|---|
| $R^1$ and $R^2$, | independently of one another, each represent alkyl, alkenyl, alkinyl, halogenoalkyl, halogenoalkenyl, halogenoalkinyl, cyanoalkyl, alkoxyalkyl, alkoxy, cycloalkyl, cycloalkylalkyl, represent aryl, aralkyl or heteroaryl, each of which is optionally substituted, or, together with the nitrogen atom to which they are bonded, represent an optionally substituted heterocycle and |
| $R^5$ | represents alkyl or cycloalkyl. |

## Revendications

1. Triazolinones substituées répondant à la formule (I)

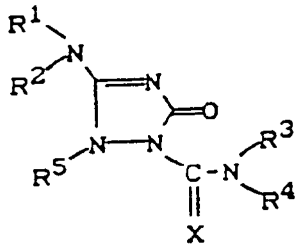

(I)

dans laquelle

$R^1$ et $R^2$ représentent chacun, indépendamment l'un de l'autre, un groupe alkyle, un groupe alcényle, un groupe alcynyle, un groupe halogénalkyle, un groupe halogénalcényle, un groupe halogénalcynyle, un groupe cyanalkyle, un groupe alcoxyalkyle, un groupe alcoxy, un groupe cycloalkyle, un groupe cycloalkylalkyle; un groupe aryle, un groupe aralkyle ou un groupe hétéroaryle, chacun de ces groupes étant éventuellement substitué; ou représentent, ensemble avec l'atome d'azote auquel ils sont liés, un composé hétérocyclique éventuellement substitué,

$R^3$ et $R^4$ représentent chacun, indépendamment l'un de l'autre, un atome d'hydrogène, un groupe alkyle, un groupe alcényle, un groupe alcynyle, un groupe halogénalkyle, un groupe halogénalcényle, un groupe halogénalcynyle, un groupe cyanalkyle, un groupe hydroxyalkyle, un groupe alcoxyalkyle, un groupe alkylaminoalkyle, un groupe dialkylaminoalkyle, un groupe alcoxycarbonylalkyle, un groupe alcoxycarbonylalcényle; un groupe cycloalkyle, un groupe cycloalkylalkyle, un groupe cycloalcényle ou un groupe cycloalcénylalkyle, chacun de ces groupes étant éventuellement substitué; un groupe hétérocyclylalkyle éventuellement substitué; un groupe aralkyle, un groupe aroyle ou un groupe aryle, chacun de ces groupes étant éventuellement substitué; un groupe alcoxy, un groupe alcényloxy, un groupe alcynyloxy, un groupe aralkyloxy ou un groupe aryloxy; ou représentent, ensemble avec l'atome d'azote auquel ils sont liés, un composé hétérocyclique éventuellement substitué,

$R^5$ représente un groupe alkyle ou un groupe cycloalkyle, et

X représente un atome d'oxygène ou un atome de soufre.

2. Triazolinones substituées répondant à la formule (I) selon la revendication 1,

dans laquelle

$R^1$ et $R^2$ représentent, indépendamment l'un de l'autre, un groupe alkyle contenant de 1 à 8 atomes de carbone, un groupe alcényle contenant de 2 à 8 atomes de carbone, un groupe alcynyle contenant de 2 à 8 atomes de carbone, un groupe halogénalkyle contenant de 1 à 8 atomes de carbone et de 1 à 17 atomes d'halogène identiques ou différents, un groupe halogénalcényle ou un groupe halogénalcynyle contenant chacun de 2 à 8 atomes de carbone et de 1 à 15 ou 13 atomes d'halogène identiques ou différents, un groupe cyanalkyle, un groupe alcoxyalkyle ou un groupe alcoxy contenant chacun de 1 à 6 atomes de carbone dans les fractions alkyle individuelles, chacun de ces groupes étant à chaîne droite ou ramifiée; un groupe cycloalkyle contenant de 3 à 7 atomes de carbone; un groupe cycloalkylalkyle contenant de 3 à 7 atomes de carbone dans la fraction cycloalkyle et de 1 à 6 atomes de carbone dans la fraction alkyle; ou encore un groupe aralkyle contenant de 6 à 10 atomes de carbone dans la fraction aryle et de 1 à 6 atomes de carbone dans la fraction alkyle, un groupe aryle contenant de 6 à 10 atomes de carbone ou un groupe hétéroaryle contenant de 2 à 9 atomes de carbone et de 1 à 3 hétéroatomes, en particulier un atome d'azote, un atome d'oxygène et/ou un atome de soufre, chacun de ces groupes étant éventuellement substitué une ou plusieurs fois de manière identique ou différente, dans lesquels, comme substituants, on envisage respectivement : un atome d'halogène, un groupe cyano, un groupe nitro; ainsi qu'un groupe alkyle, un groupe alcoxy, un groupe alkylthio, un groupe halogénalkyle, un groupe halogénalcoxy ou un groupe halogénalkylthio contenant chacun de 1 à 4 atomes de carbone et éventuellement de 1 à 9 atomes d'halogène identiques ou différents, chacun de ces groupes étant à chaîne droite ou ramifiée, ou

$R^1$ et $R^2$ représentent, ensemble avec l'atome d'azote auquel ils sont liés, un composé hétérocyclique penta- à décagonal éventuellement substitué une ou plusieurs fois de manière identique ou différente, qui peut contenir éventuellement de 1 à 2 hétéroatomes supplémentaires, en particulier un atome d'azote, un atome d'oxygène et/ou un atome de soufre, dans lequel, comme substituants, on envisage : un atome d'halogène; ainsi qu'un groupe alkyle ou un groupe halogénalkyle respectivement à chaîne droite ou ramifiée contenant chacun de 1 à 4 atomes de carbone et éventuellement de 1 à 9 atomes d'halogène identiques ou différents, ainsi que de 1 à 2 groupes oxo ou thiono,

$R^3$ et $R^4$ représentent, indépendamment l'un de l'autre, un atome d'hydrogène; un groupe alkyle contenant de 1 à 18 atomes de carbone, un groupe alcényle contenant de 2 à 8 atomes de carbone, un groupe alcynyle contenant de 2 à 8 atomes de carbone, un groupe halogénalkyle contenant de 1 à 8 atomes de carbone et de 1 à 17 atomes d'halogène identiques ou différents, un groupe halogénalcényle ou un groupe halogénalcynyle contenant chacun de 2 à 8 atomes de carbone et de 1 à 15 ou 13 atomes d'halogène identiques ou différents, un groupe cyanalkyle contenant de 1 à 8 atomes de carbone, un groupe hydroxyalkyle contenant de 1 à 8 atomes de carbone et de 1 à 6 groupes hydroxyle, un groupe alcoxyalkyle, un groupe alcoxycarbonylalkyle ou encore un groupe alcoxycarbonylalcényle contenant chacun jusqu'à 6 atomes de carbone dans les fractions alkyle ou alcényle individuelles, un groupe alkylaminoalkyle ou un groupe dialkylaminoalkyle contenant chacun de 1 à 6 atomes de carbone dans les fractions alkyle individuelles, chacun de ces groupes étant à chaîne droite ou ramifiée; ou encore représentent un groupe cycloalkyle, un groupe cycloalkylalkyle, un groupe cycloalcényle ou un groupe cycloalcénylalkyle contenant chacun de 3 à 8 atomes de carbone dans la fraction cycloalkyle ou dans la fraction cycloalcényle et éventuellement de 1 à 6 atomes de carbone dans la fraction alkyle, chacun de ces groupes étant éventuellement substitué une ou plusieurs fois de manière identique ou différente, dans lesquels, comme substituants, on envisage respectivement : un atome d'halogène, un groupe cyano; ainsi qu'un groupe alkyle ou un groupe halogénalkyle contenant chacun de 1 à 4 atomes de carbone et éventuellement de 1 à 9 atomes d'halogène identiques ou différents, chacun de ces groupes étant à chaîne droite ou ramifiée; ou encore un groupe alcanediyle ou un groupe alcènediyle, chacun comportant une double liaison et contenant chacun jusqu'à 4 atomes de carbone; en outre, $R^3$ et $R^4$ représentent, indépendamment l'un de l'autre, un groupe hétérocyclylalkyle contenant de 1 à 6 atomes de carbone dans la fraction alkyle à chaîne droite ou ramifiée et de 1 à 9 atomes de carbone, ainsi que de 1 à 3 hétéroatomes - en particulier, un atome d'azote, un atome d'oxygène et/ou un atome de soufre - dans la fraction hétérocyclyle, ce groupe étant éventuellement substitué une ou plusieurs fois de manière identique ou différente dans la fraction hétérocyclyle, dans lequel, comme substituants, on envisage : un atome d'halogène, un groupe cyano, un groupe nitro; ainsi qu'un groupe alkyle, un groupe alcoxy, un groupe alkylthio, un groupe halogénalkyle, un groupe halogénalcoxy, un groupe halogénalkylthio ou un groupe alcoxycarbonyle contenant chacun de 1 à 5 atomes de carbone et éventuellement de 1 à 9 atomes d'halogène identiques ou différents, chacun de ces groupes étant à chaîne droite ou ramifiée; en outre, $R^3$ et $R^4$ représéntent, indépendamment l'un de l'autre, un groupe alcoxy contenant de 1 à 8 atomes de carbone, un groupe alcényloxy contenant de 2 à 8 atomes de carbone ou un groupe alcynyloxy contenant de 2 à 8 atomes de carbone, chacun de ces groupes étant à chaîne droite ou ramifiée; ou encore un groupe aralkyle, un groupe aralkyloxy, un groupe aryloxy, un groupe aroyle ou un groupe aryle contenant chacun de 6 à 10 atomes de carbone dans la fraction aryle et éventuellement de 1 à 6 atomes de carbone dans la fraction alkyle, chacun de ces groupes étant éventuellement substitué une ou plusieurs fois de manière identique ou différente, dans lesquels, comme substituants du groupe aryle, on envisage, respectivement : un atome d'halogène, un groupe cyano, un groupe nitro, un groupe hydroxyle; un groupe alkyle, un groupe alcoxy, un groupe alkylthio, un groupe halogénalkyle, un groupe halogénalcoxy, un groupe halogénalkylthio, un groupe alkylsulfinyle, un groupe alkylsulfonyle, un groupe halogénalkylsulfinyle, un groupe halogénalkylsulfonyle, un groupe alcanoyle ou un groupe alcoxycarbonyle contenant chacun de 1 à 6 atomes de carbone et éventuellement de 1 à 9 atomes d'halogène identiques ou différents, chacun de ces groupes étant à chaîne droite ou ramifiée; un groupe cycloalkyle contenant de 3 à 6 atomes de carbone ou encore un groupe phénoxy, et dans lesquels, comme substituants du groupe alkyle, on envisage éventuellement : un atome d'halogène ou un groupe cyano, ou

$R^3$ et $R^4$ représentent, ensemble avec l'atome d'azote auquel ils sont liés, un composé hétérocyclique penta- à décagonal éventuellement substitué une ou plusieurs fois de manière identique ou différente, qui peut contenir éventuellement 1 à 2 hétéroatomes supplémentaires, en particulier un atome d'azote, un atome d'oxygène et/ou un atome de soufre, dans lequel, comme substituants, on envisage : un atome d'halogène; ainsi qu'un groupe alkyle ou un groupe halogénalkyle, respectivement à chaîne droite ou ramifiée, contenant chacun de 1 à 4 atomes de carbone et éventuellement de 1 à 9 atomes d'halogène identiques ou différents, ainsi que de 1 à 2 groupes oxo ou thiono,

$R^5$ représente un groupe alkyle à chaîne droite ou ramifiée contenant de 1 à 8 atomes de carbone ou un groupe cycloalkyle contenant de 3 à 8 atomes de carbone, et

X représente un atome d'oxygène ou un atome de soufre.

3. Triazolinones substituées répondant à la formule (I) selon la revendication 1, dans laquelle

$R^1$ et $R^2$ représentent chacun, indépendamment l'un de l'autre, un groupe méthyle, un groupe éthyle, un groupe n- ou i-propyle, un groupe n-, i-, s- ou t-butyle, un groupe n- ou i-pentyle, un groupe allyle, un groupe propargyle; un groupe halogénalkyle contenant de 1 à 4 atomes de carbone, un groupe halogénalcényle contenant de 3 à 6 atomes de carbone ou un groupe halogénalcynyle contenant de 3 à 6 atomes de carbone et respectivement de 1 à 9 atomes d'halogène identiques ou différents, chacun de ces groupes étant à chaîne droite ou ramifiée; un groupe cyanéthyle, un groupe méthoxyméthyle, un groupe méthoxyéthyle, un groupe diméthoxyéthyle, un groupe méthoxy, un groupe éthoxy; un groupe cyclopropyle, un groupe cyclopropylméthyle, un groupe cyclopentyle, un groupe cyclohexyle, un groupe cyclohexylméthyle, un groupe cyclohexyléthyle, un groupe cyclopentylméthyle; ou encore un groupe benzyle, un groupe phényléthyle ou un groupe phényle, chacun de ces groupes étant éventuellement substitué de 1 à 3 fois de manière identique ou différentes, dans lesquels, comme substituants, on envisage : un atome de fluor, un atome de chlore, un atome de brome, un groupe cyano, un groupe nitro, un groupe méthyle, un groupe éthyle, un groupe n- ou i-propyle, un groupe n-, i-, s- ou t-butyle, un groupe méthoxy, un groupe éthoxy, un groupe méthylthio, un groupe trifluorométhyle et un groupe trifluorométhoxy ou un groupe trifluorométhylthio, ou bien

$R^1$ et $R^2$ représentent, ensemble avec l'atome d'azote auquel ils sont liés, un composé hétérocyclique éventuellement substitué de une à trois fois de manière identique ou différente, répondant aux formules

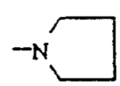 ; 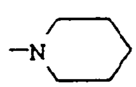 ; 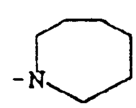 ; 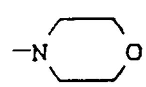 ;

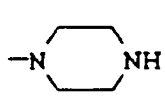 ; 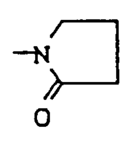 ; 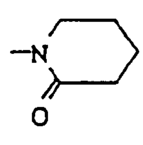 ; 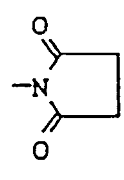 ;

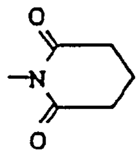 ; 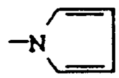 ou 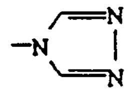

dans lequel, comme substituants, on envisage : un groupe méthyle, un groupe éthyle, un groupe n- ou i-propyle, un atome de chlore ou un groupe trifluorométhyle,

$R^3$ représente un atome d'hydrogène ou un groupe méthyle,

$R^4$ représente un groupe méthyle, un groupe éthyle, un groupe n- ou i-propyle, un groupe n-, i-, s- ou t-butyle; un groupe pentyle, un groupe hexyle, un groupe heptyle, un groupe octyle, un groupe nonyle, un groupe décyle ou un groupe dodécyle, chacun de ces groupes étant à chaîne droite ou ramifiée; un groupe allyle; un groupe butényle, un groupe pentényle, un groupe hexényle, un groupe propargyle, un groupe butynyle, un groupe pentynyle ou un groupe hexynyle, chacun de ces groupes étant à chaîne droite ou ramifiée; un groupe halogénalkyle à chaîne droite ou ramifiée contenant de 1 à 6 atomes de carbone et de 1 à 9 atomes d'halogène identiques ou différents; un groupe halogénalcényle ou un groupe halogénalcynyle, chacun de ces groupes étant à chaîne droite ou ramifiée et contenant chacun de 3 à 5 atomes de carbone et de 1 à 3 atomes d'halogène; un groupe cyanalkyle contenant de 1 à 6 atomes de carbone dans la fraction alkyle, un groupe hydroxyalkyle contenant de 1 à 6 atomes de carbone et de 1 à 3 groupes hydroxyle, un groupe alcoxyalkyle, un groupe alcoxycarbonylalkyle ou un groupe alcoxycarbonylalcényle, un groupe alkylaminoalkyle ou un groupe dialkylaminoalkyle contenant chacun jusqu'à 4 atomes de carbone dans les fractions alkyle et alcényle individuelles, chacun de ces groupes étant à chaîne droite ou ramifiée; ou encore un groupe cyclopropyle, un groupe cyclopropylméthyle, un groupe cyclopropyléthyle, un groupe cyclopentyle, un groupe cyclohexyle, un groupe cycloheptyle, un groupe cyclooctyle, un groupe cyclohexylméthyle, un groupe cyclohexyléthyle, un groupe cyclohexényle ou un groupe cyclohexénylméthyle, chacun de ces groupes étant éventuellement substitué de 1 à 3 fois de manière identique ou différente, dans lesquels, comme substituants, on envisage respectivement : un atome de fluor, un atome de chlore, un atome de brome, un groupe méthyle, un groupe éthyle, un groupe n- ou i-propyle, un groupe n-, i-, s- ou t-butyle, un groupe cyano, un groupe méthanediyle, un groupe éthanediyle, un groupe butanediyle ou un groupe butadiènediyle;

en outre, représente un groupe hétérocyclylméthyle, un groupe hétérocyclylpropyle ou un groupe hétérocyclyléthyle éventuellement substitués dans la fraction hétérocyclyle de 1 à 3 fois de manière identique ou différente, dans lesquels, comme composés hétérocycliques,

on envisage respectivement :

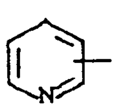 ; 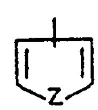 ; 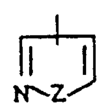 ; 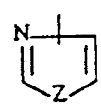 ;

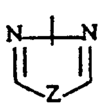 ; 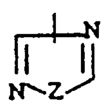 ; 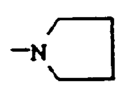 ; 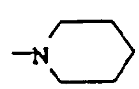 ;

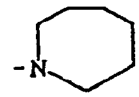 ; 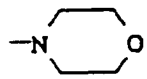 ou 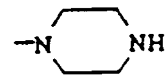

où Z représente respectivement un atome d'oxygène ou un atome de soufre, et dans lesquels, comme substituants, on envisage : un atome de fluor, un atome de chlore, un atome de brome, un groupe cyano, un groupe nitro, un groupe méthyle, un groupe éthyle, un groupe n- ou i-propyle, un groupe n-, i-, s- ou t-butyle, un groupe méthoxy, un groupe éthoxy, un groupe méthylthio, un groupe trifluorométhyle, un groupe trifluorométhoxy ou un groupe trifluorométhylthio; $R^4$ représente, en outre, un groupe alcoxy contenant de 1 à 6 atomes de carbone, un groupe alcényloxy contenant de 3 à 6 atomes de carbone ou un groupe alcynyloxy contenant de 3 à 6 atomes de carbone, chacun de ces groupes étant à chaîne droite ou ramifiée; ou encore un groupe benzyle, un groupe phényléthyle, un groupe phénylpropyle, un groupe phénylbutyle, un groupe phénylpentyle, un groupe phénylhexyle, un groupe phénylheptyle, un groupe phénylcyanométhyle, un groupe phénylcyanoéthyle, un groupe phénylcyanopropyle, un groupe benzyloxy, un groupe phényléthyloxy, un groupe phénoxy, un groupe benzoyle, un groupe phényle ou un groupe naphtyle, chacun de ces groupes étant à chaîne droite ou ramifiée et étant éventuellement substitué de 1 à 3 fois de manière identique ou différente, dans lesquels, comme substituants du groupe phényle, on envisage respectivement : un atome de fluor, un atome de chlore, un atome de brome, un groupe hydroxyle, un groupe cyano, un groupe nitro, un groupe méthyle, un groupe éthyle, un groupe n- ou i-propyle, un groupe n-, i-, s- ou t-butyle, un groupe méthoxy, un groupe éthoxy, un groupe méthylthio, un groupe trifluorométhyle, un groupe trifluorométhoxy, un groupe trifluorométhylthio, un groupe trifluorométhylsulfinyle, un groupe trifluorométhylsulfonyle, un groupe méthylsulfinyle, un groupe méthylsulfonyle, un groupe acétyle, un groupe propionyle, un groupe méthoxycarbonyle, un groupe éthoxycarbonyle, un groupe cyclohexyle ou un groupe phénoxy; ou

$R^3$ et $R^4$ représentent, ensemble avec l'atome d'azote auquel ils sont liés, un composé hétérocyclique éventuellement substitué de une à trois fois de manière identique ou différente, répondant aux formules

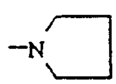 ; 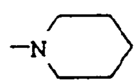 ; 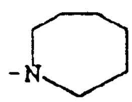 ; 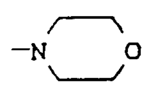 ;

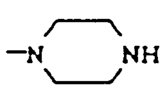 ; 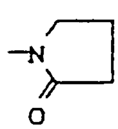 ; 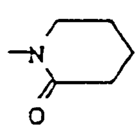 ; 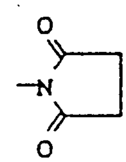 ;

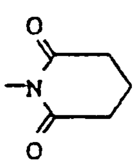 ; 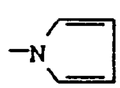 ou 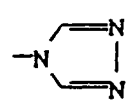

dans lequel, comme substituants, on envisage : un groupe méthyle, un groupe éthyle, un groupe n- ou i-propyle, un atome de chlore ou un groupe trifluorométhyle,

$R^5$ représente un groupe méthyle, un groupe éthyle, un groupe propyle, un groupe isopropyle, un groupe n-, i-, s- ou t-butyle, un groupe cyclopropyle, un groupe cyclobutyle, un

groupe cyclopentyle ou un groupe cyclohexyle, et
X représente un atome d'oxygène ou un atome de soufre.

**4.** Procédé pour la préparation de triazolinones substituées répondant à la formule (I)

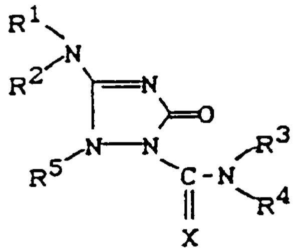

(I)

dans laquelle

$R^1$ et $R^2$ représentent chacun, indépendamment l'un de l'autre, un groupe alkyle, un groupe alcényle, un groupe alcynyle, un groupe halogénalkyle, un groupe halogénalcényle, un groupe halogénalcynyle, un groupe cyanalkyle, un groupe alcoxyalkyle, un groupe alcoxy, un groupe cycloalkyle, un groupe cycloalkylalkyle; un groupe aryle, un groupe aralkyle ou un groupe hétéroaryle, chacun de ces groupes étant éventuellement substitué; ou représentent, ensemble avec l'atome d'azote auquel ils sont liés, un composé hétérocyclique éventuellement substitué,

$R^3$ et $R^4$ représentent chacun, indépendamment l'un de l'autre, un atome d'hydrogène, un groupe alkyle, un groupe alcényle, un groupe alcynyle, un groupe halogénalkyle, un groupe halogénalcényle, un groupe halogénalcynyle, un groupe cyanalkyle, un groupe hydroxyalkyle, un groupe alcoxyalkyle, un groupe alkylaminoalkyle, un groupe dialkylaminoalkyle, un groupe alcoxycarbonylalkyle, un groupe alcoxycarbonylalcényle; un groupe cycloalkyle, un groupe cycloalkylalkyle, un groupe cycloalcényle ou un groupe cycloalcénylalkyle, chacun de ces groupes étant éventuellement substitué; un groupe hétérocyclylalkyle éventuellement substitué; un groupe aralkyle, un groupe aroyle ou un groupe aryle, chacun de ces groupes étant éventuellement substitué; un groupe alcoxy, un groupe alcényloxy, un groupe alcynyloxy, un groupe aralkyloxy ou un groupe aryloxy, chacun de ces groupes étant éventuellement substitué; ou représentent, ensemble avec l'atome d'azote auquel ils sont liés, un composé hétérocyclique éventuellement substitué,

$R^5$ représente un groupe alkyle ou un groupe cycloalkyle, et

X représente un atome d'oxygène ou un atome de soufre,

caractérisé en ce qu'on fait réagir

a) des triazolinones de formule (II)

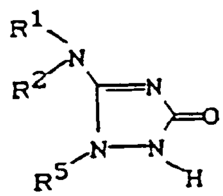

(II)

dans laquelle
$R^1$, $R^2$ et $R^5$ ont la signification indiquée ci-dessus,
et/ou des tautomères de ces composés,
avec des iso(thio)cyanates répondant à la formule générale (III)

$$R^4\text{-N=C=X} \qquad \text{(III)}$$

dans laquelle
$R^4$ et X ont la signification indiquée ci-dessus,
éventuellement en présence d'un adjuvant réactionnel et éventuellement en présence d'un diluant, ou bien en ce qu'on fait réagir

b) des chlorocarbonyl-triazolinones répondant à la formule générale (IV)

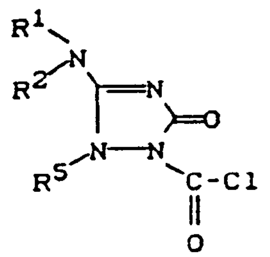

(IV)

dans laquelle
$R^1$, $R^2$ et $R^5$ ont la signification indiquée ci-dessus,
avec des amines répondant à la formule générale (V)

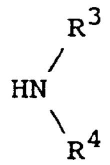

(V)

dans laquelle
$R^3$ et $R^4$ ont la signification indiquée ci-dessus,
éventuellement en présence d'un neutralisateur d'acides et éventuellement en présence d'un diluant.

5. Agents herbicides qui se caractérisent par une teneur en au moins une triazolinone substituée de formule (I) selon la revendication 1 ou 4.

6. Procédé pour lutter contre des plantes non désirées, caractérisé en ce qu'on laisse agir des triazolinones substituées de formule (I) selon la revendication 1 ou 4, sur les plantes et/ou sur leur biotope.

7. Utilisation de triazolinones substituées de formule (I) selon la revendication 1 ou 4, pour lutter contre des plantes non désirées.

8. Procédé pour la préparation d'agents herbicides, caractérisé en ce qu'on mélange des triazolinones substituées de formule (I) selon la revendication 1 ou 4, avec des diluants et/ou des substances tensioactives.

9. Chlorocarbonyl-triazolinones de formule (IV)

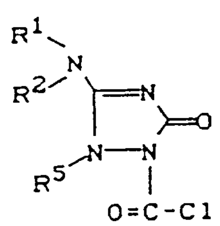

IV

dans laquelle

| | |
|---|---|
| $R^1$ et $R^2$ | représentent chacun, indépendamment l'un de l'autre, un groupe alkyle, un groupe alcényle, un groupe alcynyle, un groupe halogénalkyle, un groupe halogénalcényle, un groupe halogénalcynyle, un groupe cyanalkyle, un groupe alcoxyalkyle, un groupe alcoxy, un groupe cycloalkyle, un groupe cycloalkylalkyle; un groupe aryle, un groupe aralkyle ou un groupe hétéroaryle, chacun de ces groupes étant éventuellement substitué; ou représentent, ensemble avec l'atome d'azote auquel ils sont liés, un composé hétérocyclique éventuellement substitué, et |
| $R^5$ | représente un groupe alkyle ou un groupe cycloalkyle. |